(19) Europäisches Patentamt
European Patent Office
Office européen des brevets

(11) EP 4 644 968 A2

(12) # EUROPEAN PATENT APPLICATION

(43) Date of publication:
**05.11.2025 Bulletin 2025/45**

(21) Application number: **25175200.2**

(22) Date of filing: **15.01.2021**

(51) International Patent Classification (IPC):
***G02B 23/24*** *(2006.01)*

(52) Cooperative Patent Classification (CPC):
**A61B 18/22; G01B 11/026; G02B 23/2423; G02B 23/2469; G02B 23/2484;** A61B 2017/00066; A61B 2018/00505; A61B 2018/00547; A61B 2018/00577; A61B 2018/00589; A61B 2018/00642; A61B 2018/00702; A61B 2018/00773; A61B 2018/2075; A61B 2018/2244; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.01.2020 US 202062962001 P**
**27.11.2020 US 202063118857 P**

(62) Document number(s) of the earlier application(s) in accordance with Art. 76 EPC:
**21703332.3 / 4 069 125**

(71) Applicant: **Lumenis Ltd.**
**20692 Yokneam Illit (IL)**

(72) Inventors:
- **KHACHATUROV, Arkady**
**32495 Haifa (IL)**
- **RONDEL, Vitaly**
**3824852 Hadera (IL)**

(74) Representative: **Vossius & Partner Patentanwälte Rechtsanwälte mbB**
**Siebertstrasse 3**
**81675 München (DE)**

Remarks:
•This application was filed on 08-05-2025 as a divisional application to the application mentioned under INID code 62.
•Claims filed after the date of receipt of the application (Rule 68(4) EPC).

(54) **METHOD AND SYSTEM FOR ESTIMATING DISTANCE BETWEEN A FIBER END AND A TARGET**

(57) The present invention relates to a system for estimating a distance between an end of an optical fiber and a target, the system comprising a first laser light source having a wavelength with a first water absorption coefficient, a second laser light source having a wavelength with a second water absorption coefficient different from that of the first water absorption coefficient, a beam splitter configured to receive a portion of the laser light beams generated by the first laser light source and the second laser light source, a port optically associated with the second beam splitter, the port configured to optically couple an optical fiber with the beam splitter, wherein the laser light beams generated by the first laser light source and the second laser light source can be directed to a target via an end of the optical fiber and reflected light beams can be received via the end of the optical fiber and directed to a light detector, the light detector optically associated with the beam splitter, the light detector configured to measure intensity of the reflected light beams, and a processor and a memory comprising instructions that when executed by the processor cause the processor to estimate a distance between the end of the optical fiber and the target based on the measured intensity of the reflected light beams and the first water absorption coefficient and the second water absorption coefficient.

EP 4 644 968 A2

(52) Cooperative Patent Classification (CPC): (Cont.)
A61B 2018/2253; A61B 2090/061; A61N 2005/063

## Description

### Technical Field

The present disclosure generally relates to the field of Fiber Feedback (FFB) technology. Particularly, but not exclusively, the present disclosure relates to a method and system for estimating distance between a fiber end and a target.

### Background

Introduction of lasers into medical field and development of fiber optic technologies that use lasers have opened up wide range of applications in treatments, diagnostics, therapy and the like. Such applications range from invasive and non-invasive treatments to endoscopic surgeries and image diagnostics. For instance, in urinary stone treatment, the stones are required to be fragmented into smaller pieces. A technology known as laser lithotripsy may be used for such fragmenting process, wherein for small to medium sized urinary stones, a rigid or flexible ureteroscope is placed through the urinary tract for illumination and imaging. Simultaneously, an optical fiber is inserted through single working channel of the ureteroscope, to a target location i.e. to the location where the stone is present in the bladder, ureter, or kidney. The laser is then activated to fragment the stone into smaller pieces or to dust it. In another instance, a laser and optic fiber technology is used in coagulation or ablation treatments. During an ablation treatment, laser light is delivered to the tissue to vaporize the tissue. During a coagulation treatment, a laser is used to induce thermal damage within the tissue. Such ablation treatments may be used for treating various clinical conditions, such as Benign Prostate Hyperplasia (BPH), cancers such as prostrate cancer, liver cancer, lung cancer and the like, and for treating cardiac conditions by ablating and/or coagulating a part of the tissue in the heart.

These treatments which use laser and optic fiber technology require high amounts of accuracy to ensure that the laser is aimed at the right target (stone, tissue, tumor etc.), to achieve the clinical objective of tissue ablation, coagulation, stone fragmentation, dusting and the like. More specifically, it is of utmost importance to estimate the distance between the target and end of the optical fiber (distal end), dynamically and during a treatment, since the optimization of the laser treatment parameters, such as energy, pulse width, laser power modulation, repetition rate are also a function of the distance from the tip of the fiber and the target tissue. Therefore, the efficiency of the treatment depends upon the relative position and orientation of the optical fiber tip with respect to in the target. However, due to various factors such as movement of the optical fiber with respect to position and orientation within the body of a subject (for instance, a patient), tissue environment, movement of the tissue, surface of the target, colour of the target, optical fiber tip degradation during a laser treatment, and the like, it is extremely difficult to determine or estimate distance between the optical fiber tip and the target, when the optical fiber tip is inserted into the body of the subject. Incorrect estimation of the distance and orientation may lead to aiming the laser at a region which is not the region of interest in the target. This may lead to unnecessary complications and in some cases it may also lead to permanent damage to certain parts of the tissues, organs etc., of the subject, which could make that body part dysfunctional. In some other scenarios, incorrect distance maintenance and orientation may lead to increase in the duration of the treatment, or may lead to low quality ablation/fragmentation etc. In some cases, such as BPH or cancer, if the tumor is not ablated properly, it may lead to regrowth of such tumor leading to further complications. Therefore, it is extremely important to maintain accurate distance between the optical fiber tip and the target, while performing certain treatments using laser and optical fiber technology as discussed above.

One of the existing techniques provides a method of estimating distance between a target tissue and distal end of an optical fiber by measuring and comparing intensity values of reflections of the light beams, wherein the light beams are transmitted through the optical fiber by modulating numerical aperture of the light beams. However, it is not always convenient to shift the numerical apertures of the light beams. Moreover, separation of the reflection of light beams of different numerical apertures, required for distance estimation, may be difficult.

Therefore, there exists a need to provide an efficient method and a system to accurately estimate distance between the distal end of the optical fiber and the target.

### Summary

The present disclosure discloses a Fiber Feedback (FFB) technique, that provides a method and system for estimating distance between a fiber end and a target. The method includes illuminating, by a Light Emitting, Transmitting and Detecting (LETD) system, the target with laser light of different wavelengths having low and high water absorption coefficients, using different laser light sources. The wavelengths may be selected in such a way that, they are close to each other and belong to the same "nm scale". Further, the LETD system receives returned signal corresponding to the incident laser light of different wavelengths. The returned signal comprises light beams reflected from the target post illumination. The one or more light detectors configured in the LETD system may detect the returned signal to measure intensity values of the returned signal of a specific wavelength. Using the measured intensity values, a processing unit may then estimate the distance between the fiber end and the target. The present disclosure uses LETD system in different configurations comprising different arrangements of various optical components such as beam combiners, beam splitters, polarizers, collimators, Wave Division Multiplexers (WDM), light detectors and the like. The present disclosure enables accurate estimation of distance between fiber end and the target, and also provides a robust distance estimation technique which is compatible with different types of targets. Further, the present disclosure may be used for the purpose of smart lasing. For instance, during the treatment, the target may move around, back and forth or otherwise, or may change its shape and size. Therefore, parameters for the laser sources that are pre-set before initiating lasing on the target, may become less effective. Conventionally, such pre-set parameters are manually changed, which may be error prone and time consuming, or in some cases the pre-set parameters may be left unchanged which may lead to scenarios where the optical fiber may be too close or too far from the target. Therefore, the present disclosure allows automatic and real-time monitoring of the distance between the optical fiber and the target, and enables automatic changing of the lasing pre-set parameters to adjust the lasing in accordance with the target shape, position etc., for best results.

In an aspect, a system for treating skin tissue with a source of treatment light accurately estimating a distance between a fiber end and a target, the system comprising: an optical fiber, the optical fiber configured to deliver laser light beams originating from a plurality of laser light sources to a target, and is configured to deliver laser light beams reflected from the target to one or more light detectors; a Light Emitting, Transmitting and Detecting (LETD); and a processing unit. The Light Emitting, Transmitting and Detecting (LETD) system comprising:

(i) the plurality of laser light sources comprising a first polarized laser source (L1) and a second polarized laser source (L2) having a wavelength with water absorption coefficient different from that of the first polarized laser source (L1), and one or more other laser sources, the plurality of laser light sources configured to generate incident laser light beams for treating the target;
(ii) a first beam splitter configured to: receive incident laser light beams from the first polarized laser source (L1) and the second polarized laser source (L2); and align the incident laser light beams along a single optical path;
(iii) a polarizer configured to receive the aligned incident laser light beams from the first beam splitter, and to output polarized laser light beams;
(iv) a first beam combiner;
(v) a second beam splitter;
(vi) a polarized beam splitter;
(vii) a first light detector; and
(viii) a second light detector.

The first beam combiner configured to:

(a) receive the polarized laser light beams from the polarizer;
(b) combine the polarized laser light beams with an aiming beam and a treatment beam received from the one or more other laser sources; and
(c) output combined laser light beams.

The second beam splitter configured to:

(a) receive the combined laser light beams from the first beam combiner;
(b) align the combined laser light beams along a single optical path;
(c) deliver the aligned combined laser light beams of the second beam splitter to the target via the optical fiber;
(d) receive reflected light via the optical fiber, upon delivering the aligned combined laser light beams of the second beam splitter to the target;
(e) align laser light beams of the reflected light along a single optical path; and
(f) transmit the aligned laser light beams of the reflected light to a polarized beam splitter.

The polarized beam splitter configured to: receive the aligned laser light beams of the reflected light from the second beam splitter; and split the aligned laser light beams of the reflected light into reflected S-Polarized and transmitted P-polarized beams.

The first light detector configured to: detect and measure intensity of the transmitted P-polarized beams of the reflected light; and transmit the measured intensity of the transmitted P-polarized beams of the reflected light to a processing unit associated with the LETD system.

The second light detector configured to: detect and measure intensity of the reflected S-polarized beams of the reflected light; and transmit the measured intensity of the reflected S-polarized beams of the reflected light to the processing unit.

The processing unit configured to: receive the measured intensities of the transmitted P-polarized beams and reflected S-polarized beams of the reflected light from the first light detector and the second light detector, respectively; and estimate a distance between a distal end of the optical fiber and the target based on the measured intensities, the water absorption coefficients of the respective wavelengths of the plurality of laser light sources, and a target reflection coefficient.

In another aspect, the system further comprises a power detector associated with the first beam splitter configured to measure optical power of the incident laser light beams generated by the first polarized laser source (L1) and the second polarized laser source (L2). Also, the system further comprises an indicator associated with the processing unit configured to indicate the estimated distance between the distal end of the optical fiber and the target, wherein the indicator comprises at least one of visual indicator, audio indicator and a haptic indicator. The system, wherein the wavelength of the first laser light source has a higher water absorption coefficient than the wavelength of the second laser light source. The system, wherein the wavelength of the first laser light source and second laser light source are predefined, and are selected such that the wavelengths are proximal on a wavelength scale. The system, wherein the laser light beams of the reflected light comprises at least one of light beams reflected from the target, light beams reflected from region around the target, and light beams reflected from the distal end of the optical fiber.

In a further aspect, a system for accurately estimating a distance between a fiber end and a target, the system comprising: an optical fiber; a Light Emitting, Transmitting and Detecting (LETD); and a processing unit. The Light Emitting, Transmitting and Detecting (LETD) system comprising:

(i) the plurality of laser light sources comprising a first polarized laser source (L1) and a second polarized laser source (L2) having a wavelength with water absorption coefficient different from that of the first polarized laser source (L1), and one or more other laser sources, the plurality of laser light sources configured to generate laser light beams for treating the target;
(ii) a second beam combiner configured to: receive incident laser light beams from the first polarized laser source (L1) and the second polarized laser source (L2); and combine the incident laser light beams;
(iii) a polarizer configured to: receive the combined incident laser light beams from the second beam combiner, and output polarized laser light beams;
(iv) a first beam combiner;
(v) a second beam splitter;
(vi) a polarized beam splitter;
(vii) a first light detector configured to: detect and measure intensity of the transmitted P-polarized beams of the reflected light; and transmit the measured intensity of the transmitted P-polarized beams of the reflected light to a processing unit associated with the LETD system; and
(viii) a second light detector.

The optical fiber configured to: deliver laser light beams originating from a plurality of laser light sources to a target, and deliver laser light beams reflected from the target to one or more light detectors.

The first beam combiner configured to:

(a) receive the polarized laser light beams from the polarizer ;
(b) combine the polarized laser light beams with an aiming beam and a treatment beam received from the one or more other laser sources; and
(c) output combined laser light beams.

The second beam splitter configured to:

(a) receive the combined laser light beams from the first beam combiner
(b) align the combined laser light beams along a single optical path;
(c) deliver the aligned combined laser light beams of the second beam splitter to the target via the optical fiber;
(d) receive reflected light, via the optical fiber, upon delivering the aligned combined laser light beams of the second beam splitter to the target; and
(e) align laser light beams of the reflected light along a single optical path, and transmit the aligned laser light beams of the reflected light to a polarized beam splitter.

The polarized beam splitter configured to: receive the aligned laser light beams of the reflected light from the second beam splitter; and split the aligned laser light beams of the reflected light into reflected S-Polarized and transmitted P-polarized beams.

The second light detector configured to: detect and measure intensity of the reflected S-polarized beams of the reflected light; and transmit the measured intensity of the reflected S-polarized beams of the reflected light to the processing unit.

The processing unit configured to: receive the measured intensities of the transmitted P-polarized beams and reflected S-polarized beams of the reflected light from the first light detector and the second light detector, respectively; and estimate a distance between a distal end of the optical fiber and the target based on the measured intensities, the water absorption coefficients of the respective wavelengths of the plurality of laser light sources, and a target reflection coefficient.

The system further comprises a power detector associated with the second beam splitter configured to measure optical power of the combined laser light comprising the incident laser light beams generated by the first polarized laser source (L1), the second polarized laser source (L2), and the one or more other lasers. The system further comprises an indicator associated with the processing unit, wherein the indicator is configured to indicate the estimated distance between the distal end of the optical fiber and the target, wherein the indicator comprises at least one of visual indicator, audio indicator and a haptic indicator. The system, wherein the wavelength of the first laser light source has a higher water absorption coefficient than the wavelength of the second laser light source. The system, wherein the wavelength of the first laser light source and second laser light source are predefined, and are selected such that the wavelengths are proximal on a wavelength scale. The system, wherein the laser light beams of the reflected light comprises at least one of light beams reflected from the target, light beams reflected from region around the target, and light beams reflected from the distal end of the optical fiber.

In another aspect, there is of a system for accurately estimating a distance between a fiber end and a target, the system comprising:

an optical fiber;
a processing unit; and
a Light Emitting, Transmitting and Detecting (LETD) system comprising:

(i) the plurality of laser light sources comprising a first non-polarized laser source (L1’) and a second non-polarized laser source (L2’) having a wavelength with water absorption coefficient different from that of the first non-polarized laser source (L1’), and one or more other laser sources, the plurality of laser light sources configured to generate incident laser light beams for treating the target;
(ii) a first beam splitter;
(iii) a first beam combiner;
(iv) a second beam splitter; and
(v) a third light detector.

The optical fiber configured to: deliver laser light beams originating from a plurality of laser light sources, to a target; and deliver laser light beams reflected from the target, to one or more light detectors.

The first beam splitter configured to: receive incident laser light beams from the first non-polarized laser source (L1’) and the second non-polarized laser source (L2’); and align the incident laser light beams along a single optical path.

The first beam combiner configured to:

(a) receive the aligned incident laser light beams from the first beam splitter;
(b) combine the aligned incident laser light beams with an aiming beam and a treatment beam received from the one or more other laser sources; and
(c) output combined laser light beams.

The second beam splitter configured to:

(a) receive the combined laser light beams from the first beam combiner;
(b) align the combined laser light beams along a single optical path;
(c) deliver the aligned combined laser light beams of the second beam splitter to the target via the optical fiber;
(d) receive reflected light, via the optical fiber, upon delivering the aligned combined laser light beams of the second beam splitter to the target; and
(e) align laser light beams of the reflected light along a single optical path, and transmit the aligned laser light beams of the reflected light to a third light detector.

The third light detector configured to: detect and measure intensity of the aligned laser light beams of the reflected light; and transmit the measured intensity to a processing unit associated with the LETD system.

The processing unit configured to: receive the measured intensity of the aligned laser light beams of the reflected light from the third light detector; and estimate a distance between a distal end of the optical fiber and the target based on the measured intensity, the water absorption coefficients of the respective wavelengths of the plurality of laser light sources, and a target reflection coefficient.

In a further aspect, the system comprises a power detector associated with the first beam splitter configured to measure optical power of the laser light beams generated by the first non-polarized laser source (L1') and the second non-polarized laser source (L2'). The system further comprises an indicator associated with the processing unit, wherein the indicator is configured to indicate the estimated distance between the distal end of the optical fiber and the target, wherein the indicator comprises at least one of visual indicator, audio indicator and a haptic indicator. The system, wherein the wavelength of the first laser light source has a higher water absorption coefficient than the wavelength of the second laser light source. The system, wherein the wavelength of the first laser light source and second laser light source are predefined, and are selected such that the wavelengths are proximal on a wavelength scale. The system, wherein the laser light beams of the reflected light comprises at least one of light beams reflected from the target, light beams reflected from region around the target, and light beams reflected from a proximal end and the distal end of the optical fiber.

In one aspect, there is a system for accurately estimating a distance between a fiber end and a target, the system comprising:

a processor;
an optical fiber; and
a Light Emitting, Transmitting and Detecting (LETD) system comprising:

(i) a plurality of laser light sources;
(ii) a first beam splitter;
(iii) a third beam splitter;
(iv) a polarizer;
(v) a first beam combiner;
(vi) a second beam splitter;
(vii) a polarized beam splitter;
(viii) a first light detector; and
(ix) a second light detector.

The optical fiber configured to: deliver laser light beams originating from a plurality of laser light sources to a target; and deliver laser light beams reflected from the target, to one or more light detectors.

The plurality of laser light sources comprising:

(a) a first polarized laser source (L1);
(b) a second polarized laser source (L2) having a wavelength with water absorption coefficient different from that of the first polarized laser source (L1);
(c) a third polarized laser source (L3) having a wavelength with water absorption coefficient different from that of the first polarized laser source (L1) and the second polarized laser source (L2); and
(d) one or more other laser sources, wherein the plurality of laser light sources are configured to generate incident laser light beams for treating the target.

The first beam splitter configured to: receive incident laser light beams from the first polarized laser source (L1), the second polarized laser source (L2), and the third polarized laser source (L3); and align the incident laser light beams along a single optical path.

The third beam splitter configured to: receive the aligned incident laser beams from the first beam splitter; and align the aligned incident laser light beams of the first beam splitter and the incident laser light beams received from the third polarized laser source (L3) along a single optical path.

The polarizer configured to: receive the aligned incident laser light beams from the third beam splitter; and output the polarized laser light.

The first beam combiner configured to:

(a) receive the polarized laser light beams from the polarizer;
(b) combine the polarized laser light beams with an aiming beam and a treatment beam received from the one or more other laser sources; and
(c) output combined laser light beams.

The second beam splitter configured to:

(a) align the combined laser light beams from the first beam combiner;
(b) align the combined laser light beams along a single optical path;
(c) deliver the aligned combined laser light beams of the second beam splitter to the target via the optical fiber;
(d) receive reflected light, via the optical fiber, upon delivering the aligned combined laser light beams of the second beam splitter to the target;
(e) align laser light beams of the reflected light along a single optical path; and
(f) transmit the aligned laser light beams of the reflected light to a polarized beam splitter.

The polarized beam splitter configured to split the aligned laser light beams of the reflected light into reflected S-Polarized and transmitted P-polarized beams.
The first light detector configured to: detect and measure intensity of the transmitted P-polarized beams of the reflected light; and transmit the measured intensity of the transmitted P-polarized beams of the reflected light to a processing unit associated with the LETD system.
The second light detector configured to: detect and measure intensity of the reflected S-polarized beams of the reflected light; and transmit the measured intensity of the reflected S-polarized beams of the reflected light to the processing unit.
The processing unit configured to: receive the measured intensities of the transmitted P-polarized beams and reflected S-polarized beams of the reflected light from the first light detector and the second light detector, respectively; and estimate a distance between a distal end of the optical fiber and the target based on the measured intensities, the water absorption coefficients of the respective wavelengths of the plurality of laser light sources, and a target reflection coefficient.
In yet a further aspect of the system, the system further comprises a power detector associated with the third beam splitter configured to measure optical power of the laser light beams generated by the first polarized laser source (L1), the second polarized laser source (L2) and the third polarized laser source (L3). The system that further comprises an indicator associated with the processing unit, wherein the indicator is configured to indicate the estimated distance between the distal end of the optical fiber and the target, wherein the indicator comprises at least one of visual indicator, audio indicator and a haptic indicator. The system, wherein the wavelength of the first laser light source (L1) has a higher water absorption than the wavelength of the second laser light source (L2), and the wavelength of the third laser light source (L3) has a higher water absorption than the wavelength of the first laser light source (L1) and the second laser light source (L2). The system, wherein the wavelength of the first laser light source and second laser light source are predefined and are selected such that the wavelengths are proximal on a wavelength scale. The system, wherein the laser light beams of the reflected light comprises at least one of light beams reflected from the target, light beams reflected from region around the target, and light beams reflected from a proximal end and the distal end of the optical fiber.
In one aspect, there is a system for accurately estimating a distance between a fiber end and a target, the system comprising:

an optical fiber;
a processor;
a Light Emitting, Transmitting and Detecting (LETD) system.

The Light Emitting, Transmitting and Detecting (LETD) system comprising:

(i) the plurality of laser light sources comprising a first polarized laser source (L1), a second polarized laser source (L2) having a wavelength with water absorption coefficient different from that of the first polarized laser source (L1), a third polarized laser source (L3) having a wavelength with water absorption coefficient different from that of the first polarized laser source (L1) and the second polarized laser source (L2), and one or more other laser sources, the plurality of laser light sources configured to generate incident laser light beams for treating the target;
(ii) a Wavelength Division Multiplexer (WDM);
(iii) a fourth beam splitter;
(iv) a collimator;
(v) a first beam combiner;
(vi) a second beam splitter;
(vii) a third light.

The optical fiber configured to: deliver laser light beams originating from a plurality of laser light sources to a target; and laser light beams reflected from the target to one or more light detectors.
The WDM configured to: receive incident laser light beams from the first polarized laser source (L1), the second polarized laser source (L2), the third polarized laser source (L3), and an aiming beam generated by the one or more other laser sources; and align the incident laser light beams along a single optical path.

The fourth beam splitter configured to: receive the aligned incident laser light beams from the WDM; and align incident laser light beams of the WDM along a single optical path.

The collimator configured to: receive the aligned incident laser light beams from the fourth beam splitter; and narrow down the aligned output laser light beams into parallel laser light beams.

The first beam combiner configured to:

(a) receive the parallel laser light beams from the polarizer;
(b) combine the parallel light beams with another aiming beam and a treatment beam received from the one or more other laser sources; and
(c) output combined laser light beams.

The second beam splitter configured to:

(a) receive the combined laser light beams;
(b)align the combined laser light beams along a single optical path;
(c) deliver the aligned combined laser light beams of the second beam splitter to the target via the optical fiber;
(d) receive reflected light, via the optical fiber, upon delivering the aligned combined laser light beams of the second beam splitter to the target;
(e) align laser light beams of the reflected light along a single optical path; and
(f) transmit the aligned light beams of the reflected light to a third light detector.

The third light detector configured to: detect and measure intensity of the aligned laser light beams of the reflected light; and transmit the measured intensity of the aligned laser light beams of the reflected light to a processing unit associated with the LETD system.

The processing unit configured to: receive the measured intensity of the aligned laser light beams of the reflected light; and estimate a distance between a distal end of the optical fiber and the target based on the measured intensity, the water absorption coefficients of the respective wavelengths of the plurality of laser light sources, and a target reflection coefficient.

In yet a further aspect, the system further comprises a power detector associated with the fourth beam splitter configured to measure optical power of the laser light beams generated by the first polarized laser source (L1), the second polarized laser source (L2), the third polarized laser source (L3) and the one or more other laser sources. The system further comprises an indicator associated with the processing unit, wherein the indicator is configured to indicate the estimated distance between the distal end of the optical fiber and the target, wherein the indicator comprises at least one of visual indicator, audio indicator and a haptic indicator.

The system, wherein the wavelength of the first laser light source (L1) has a higher water absorption coefficient than the wavelength of the second laser light source (L2), and the wavelength of the third laser light source (L3) has a higher water absorption co-efficient than the wavelength of the first laser light source (L1) and the second laser light source (L2). The system, wherein the wavelength of the first laser light source (L1), the second laser light source (L2) and the third laser light source (L3) are predefined and are selected such that the wavelengths are proximal on a wavelength scale. The system, wherein the laser light beams of the reflected light comprises at least one of light beams reflected from the target, light beams reflected from region around the target, and light beams reflected from a proximal end and the distal end of the optical fiber.

An aspect of a system for accurately estimating a distance between a fiber end and a target, the system comprising:

a processor;
an optical fiber; and
a Light Emitting, Transmitting and Detecting (LETD) system.

The Light Emitting, Transmitting and Detecting (LETD) system comprising:

(i) the plurality of laser light sources comprising a first polarized laser source (L1), a second polarized laser source (L2) having a wavelength with water absorption coefficient different from that of the first polarized laser source (L1), a third polarized laser source (L3) having a wavelength with water absorption coefficient different from that of the first polarized laser source (L1) and the second polarized laser source (L2), and one or more other laser sources, the plurality of laser light sources configured to generate incident laser light beams for treating the target;
(ii) a Wavelength Division Multiplexer (WDM);

(iii) a fourth beam splitter;
(iv) a circulator;
(v) a collimator; and
(vi) a first beam combiner.

The optical fiber configured to deliver laser light beams originating from a plurality of laser light sources to a target, and laser light beams reflected from the target to one or more light detectors.

The Wavelength Division Multiplexer (WDM) configured to: receive incident laser light beams from the first polarized laser source (L1), the second polarized laser source (L2), the third polarized laser source (L3), and an aiming beam generated by the one or more other laser sources; and to align the incident laser light beams along a single optical path.

The fourth beam splitter configured to: receive the aligned incident laser light beams from the WDM; and align incident laser light beams of the WDM along a single optical path.

The circulator configured to: receive the aligned incident laser light beams from the fourth beam splitter; and enable the aligned incident laser light beams to travel in a single direction.

The collimator configured to: receive the aligned incident laser light beams from the circulator; and narrow down the aligned laser light beams received from the circulator into parallel laser light beams.

The first beam combiner configured to:

(a) receive the parallel laser light beams from the polarizer;
(b) to combine the parallel light beams with another aiming beam and a treatment beam received from the one or more other laser sources;
(c) output combined laser light beams; and
(d) deliver the combined laser light beams of the first beam combiner to the target via the optical fiber.

The circulator further configured to:

(a) receive reflected light, via the optical fiber, upon delivering the combined laser light beams of the first beam combiner to the target;
(b) enable the laser light beams of the reflected light to travel in a single direction; and
(c) transmit laser light beams of the reflected light coming from the collimator, to a third light detector.

The third light detector configured to: detect and measure intensity of the aligned laser light beams of the reflected light received from the collimator; and transmit the measured intensity of the aligned laser light beams of the reflected light to a processing unit associated with the LETD system.

The processing unit configured to: receive the measured intensity of the aligned laser light beams of the reflected light; and estimate a distance between a distal end of the optical fiber and the target based on the measured intensity, the water absorption coefficients of the respective wavelengths of the plurality of laser light sources, and a target reflection coefficient.

The system further comprises a power detector associated with the fourth beam splitter configured to measure optical power of the laser light beams generated by the first polarized laser source (L1), the second polarized laser source (L2), the third polarized laser source (L3) and the one or more other laser sources. The system further comprises an indicator associated with the processing unit, wherein the indicator is configured to indicate the estimated distance between the distal end of the optical fiber and the target, wherein the indicator comprises at least one of visual indicator, audio indicator and a haptic indicator.

The system, wherein the wavelength of the first laser light source (L1) has a higher water absorption coefficient than the wavelength of the second laser light source (L2), and the wavelength of the third laser light source (L3) has a higher water absorption co-efficient than the wavelength of the first laser light source (L1) and the second laser light source (L2). The system, wherein the wavelength of the first laser light source (L1), the second laser light source (L2) and the third laser light source (L3) are predefined and are selected such that the wavelengths are proximal on a wavelength scale. The system, wherein the laser light beams of the reflected light comprises at least one of light beams reflected from the target, light beams reflected from region around the target, and light beams reflected from a proximal end and the distal end of the optical fiber. The system, wherein the third polarized laser source (L3) is configured for calibration of an optical fiber condition. The system, wherein the optical fiber condition is the optical quality of the distal end of the optical fiber. The system, wherein the calibration is done in real time, and a deviation from the calibration indicates a degradation in the optical condition of the distal end of the optical fiber.

The system further comprising a second indicator associated with the processing unit configured to indicate to a user a degradation of the optical condition of the distal end of the optical fiber. The system, wherein the proximal end of the optical fiber is coated with an anti-reflective coat to reduce noise caused at the proximal end of the optical fiber and to increase dynamic range of the reflected light received from the target. The system, wherein the proximal end of the optical fiber is cut at a predefined angle to enable diversion of unwanted reflected light from the proximal end of the optical fiber. The system, wherein the first beam combiner is configured to receive from the one or more other laser sources at least one non-polarized treatment beam and to output the combined laser light beam.

In an additional aspect there is method of estimating distance between a fiber end and a target, the method comprising:

providing a Light Emitting, Transmitting and Detecting (LETD) system comprising a plurality of laser light sources and a plurality of light detectors;
providing a processing unit;
illuminating, by a LETD system, a target with incident laser light beams of plurality of wavelengths, via an optical fiber, using at least one of the plurality of laser light sources;
receiving, by the LETD system, via the optical fiber and the plurality of light detectors, reflected light from the target;
measuring, by the LETD system, via the light detectors, intensities of light beams of the reflected light of each of the plurality of wavelengths;
transmitting, by the LETD system, the measured intensities to a processing unit,
wherein the processing unit receives the measured intensities of the light beams of the reflected light of each of the plurality of wavelengths, from the LETD system, and estimates a distance between a distal end of the optical fiber and the target based on the measured intensities, a water absorption coefficients of the respective plurality of wavelengths, and a target reflection coefficient.

In yet another aspect, there is a method of estimating distance between a fiber end and a target, the method comprising:

providing a Light Emitting, Transmitting and Detecting (LETD) system comprising a plurality of laser light sources having a plurality of wavelengths and a plurality of light detectors;
providing a processing unit;
receiving, by the processing unit, measured intensities of the light beams of the reflected light of each of the plurality of wavelengths, from the LETD system, wherein the LETD system is configured to:

(i) illuminate a target with incident laser light beams of plurality of wavelengths, via an optical fiber, using at least one of the plurality of laser light sources; and
(ii) measure intensities of light beams of the reflected light of each of the plurality of wavelengths;

estimating, by the processing unit, a distance between a distal end of the optical fiber and the target based on the measured intensities, a water absorption coefficients of the respective plurality of wavelengths, and a target reflection coefficient.

The method, wherein the plurality of laser light sources comprises a first polarized laser source (L1) having a wavelength with a high water absorption coefficient (HI), a second polarized laser source (L2) with a low water absorption coefficient (LO), a third polarized laser source (L3) having a wavelength with a higher water absorption coefficient than the first polarized laser source (L1), a first non-polarized laser source (L1') having a wavelength with the high water absorption coefficient (HI), a second non-polarized laser source (L2') with the low water absorption coefficient (LO), a third non-polarized laser source (L3') having the wavelength with the higher water absorption coefficient than the first non-polarized laser source (L1'), and one or more other laser sources.

The method, wherein: the water absorption coefficient of the first polarized laser source (L1) and the first non-polarized laser source (L1') is higher than the water absorption coefficient of the second polarized laser source (L2) and the second non-polarized laser source (L2'); and the water absorption co-efficient of the third polarized laser source (L3) and the third non-polarized laser source (L3') is higher than the water absorption coefficient of the first polarized laser source (L1) and the first non-polarized laser source (L1'), and the water absorption coefficient of the second polarized laser source (L2) and the second non-polarized laser source (L2').

The method, wherein estimating the distance between the distal end of the optical fiber and the target comprises: determining, by the processing unit, a ratio of the measured intensities of the light beams of the reflected light belonging to two different wavelengths of the plurality of wavelengths, wherein the two different wavelengths belong to one of a first polarized laser source (L1) and a second polarized laser source (L2), or a first non-polarized laser source (L1') and a second non-polarized laser source (L2'), using an equation:

$$\frac{I_{(HI)}}{I_{(LO)}} = \frac{R}{R} * e^{(\lambda_{LO} - \lambda_{HI}) * X}$$

wherein,

$I_{(HI)}$ and $I_{(LO)}$ are the measured intensities of light beams corresponding to the two different wavelengths respectively,

$\frac{I_{(HI)}}{I_{(LO)}}$ the ratio of the measured intensities of the light beams of the reflected light belonging to the two different wavelengths,
**R** is the target reflection coefficient,
$\lambda_{LO}$ and $\lambda_{HI}$ are water absorption coefficients of the two different wavelengths respectively,
**X** is the distance between the distal end of the optical fiber and the target; and
estimating, by the processing unit, the distance **(X)** between the distal end of the optical fiber and the target based on the ratio of the measured intensities ($I_{(HI)}$ and $I_{(LO)}$) of the two different wavelengths, the water absorption coefficients of the two different wavelengths ($\lambda_{LO}$ and $\lambda_{HI}$), and the target reflection coefficient **(R)** using the equation:

$$X = \frac{\ln(\frac{I_{(HI)}}{I_{(LO)}})}{\lambda_{LO} - \lambda_{HI}}$$

wherein "In" is a natural logarithm.

The method further comprises indicating, by an indicator associated with the processing unit, the estimated distance between the distal end of the optical fiber and the target, wherein the indicator comprises at least one of a visual indicator, an audio indicator and a haptic indicator. The method further comprises measuring, by a power detector, an optical power of the laser light beams generated by the plurality of laser light sources. The method, wherein the third polarized laser source (L3) and the third non-polarized laser source (L3') are configured for calibration of an optical fiber condition in real-time. The method, wherein the methods are performed using at least one of the systems.

The method, wherein calibrating the optical fiber condition comprises:

(a) receiving, by the processing unit from the LETD system measured intensities of a light beams of reflected light belonging to each of the plurality of wavelengths;
(b) determining, by the processing unit, a calibration factor based on the measured intensity ($\mathbf{IR_{(CAL)}}$) of the light beams of the reflected light belonging to the wavelength of the third polarized laser light source (L3) or a third non-polarized laser light source (L3'), implemented under the "no target condition", and the measured intensity ($\mathbf{I_{(CAL)}}$) of the light beams of the reflected light of the wavelength belonging to the third polarized laser light source (L3) or the third non-polarized laser light source (L3') determined; and
(c) estimating, by the processing unit, distance between a distal end of the optical fiber and the target based on the measured intensities, water absorption coefficients of the respective wavelengths of the plurality of laser light sources, the determined calibration factor and a target reflection coefficient, thereby calibrating the optical fiber condition,

wherein the LETD system is configured to:

(i) illuminate a target under a "no target condition" with incident laser light beams of a plurality of wavelengths, via an optical fiber, using at least one of, a plurality of laser light sources and one or more other laser sources, for the "no target condition";
(ii) receive the reflected light of incident laser light beams of each of a plurality of wavelengths, via the optical fiber; and
(iii) measure intensities of the light beams of the reflected light belonging to each of the plurality of wavelengths, wherein the measured intensities are transmitted to a processing unit.

The method above further comprising receiving, by the processing unit from the LETD system measured intensities of light beams belonging to each of at least three of the plurality of wave lengths of reflected light under a "no target condition". Wherein the LETD system is configured to:

(i) illuminate a target under a "no target condition" with incident laser light beams of a plurality of wavelengths, via an optical fiber, using at least three of a plurality of laser light sources;
(ii) receive the reflected light of incident laser light beams of each of at least three of a plurality of wavelengths, via the optical fiber;
(iii) measure intensities of the reflected light of incident laser light beams of each at least three of a plurality of wavelengths;
(iv) transmit the measured intensities of the reflected light of incident laser light beams of each at least three of a plurality of wavelengths to the processing unit.

After receiving, storing, by the processing unit, the measured intensities of the reflected light of incident laser light beams of each at least three of a plurality of wavelengths as an internal reflection pre-treatment value (IR cal-pre).

The method further comprising after storing the internal reflection pre-treatment value, receiving, by the processing unit from the LETD system, new measured intensities of light beams of reflected light belonging to each of the plurality of wavelengths. Determining, by the processing unit, a calibration factor based on at least one of the following:

(i) the stored internal reflection pre-treatment value ($IR_{CAL\text{-}PRE}$) of a third polarized laser light source (L3), and the new measured intensity (**$IR_{CAL\text{-}DUR}$**) of the light beams of the reflected light of the wavelength belonging to the third polarized laser light source; or
(ii) the stored internal reflection pre-treatment value ($IR_{\mathbf{CAL\text{-}PRE}}$) of a third non-polarized laser light source (L3'), and the new measured intensity **($IR_{CAL\text{-}DUR}$)** of the light beams of the reflected light of the wavelength belonging to the third non-polarized laser light source (L3').

Determining an optical fiber condition comprises: estimating, by the processing unit, distance between a distal end of the optical fiber and the target based on the measured intensities, water absorption coefficients of the respective wavelengths of the plurality of laser light sources, the determined calibration factor and a target reflection coefficient, thereby calibrating the optical fiber condition.

The method, wherein the calibration factor is determined using the equation: $\mathrm{CF} = \frac{I_{(CAL)}}{IR_{(CAL)}}$ wherein, CF is the calibration factor.

The method, wherein estimating distance between the distal end of the optical fiber and the target comprises:

determining, by the processing unit, new calibrated intensities of the light beams of the reflected light belonging to two different wavelengths of the plurality of wavelengths, based on the measured intensities of the light beams of the reflected light belonging to the two different wavelengths of the plurality of wavelengths, wherein the two different wavelengths belong to one of a first polarized laser source (L1) and a second polarized laser source (L2), or a first non-polarized laser source (L1') and a second non-polarized laser source (L2'), using the equation:

$$\mathbf{I'' = I - IR \times CF}$$

wherein, **I"** is the new calibrated intensity of the light beams of the reflected light of a particular wavelength, **I** is the measured intensity of the light beams of the reflected light of the particular wavelength, CF is the determined calibration factor and **IR** is the intensity of the light beams of the reflected light of the particular wavelength measured for the "no target condition";
determining, by the processing unit, a ratio of the new calibrated intensities of the light beams of the reflected light belonging to the two different wavelengths of the plurality of wavelengths, using an equation:

$$\frac{I''_{(HI)}}{I''_{(LO)}} = \frac{R}{R} * e^{(\lambda_{LO} - \lambda_{HI}) * X}$$

wherein,

$I''_{(HI)}$ and $I''_{(LO)}$ are the new calibrated intensities of light beams corresponding to the two different wavelengths

respectively,

$\frac{I''_{(HI)}}{I''_{(LO)}}$ the ratio of the new calibrated intensities of the light beams of the reflected light belonging to the two different wavelengths,

**R** is the target reflection coefficient,

$\lambda_{LO}$ and $\lambda_{HI}$ are water absorption coefficients of the two different wavelengths respectively,

**X** is the distance between the distal end of the optical fiber and the target; and

estimating, by the processing unit, the distance between the distal end of the optical fiber and the target **(X)** based on the ratio of the new calibrated intensities (***I*"**$_{(HI)}$ and ***I*"**$_{(LO)}$) of the two different wavelengths, the water absorption coefficients of the two different wavelengths ($\lambda_{LO}$ and $\lambda_{HI}$), and the target reflection coefficient **(R)** using the equation:

$$X = \frac{\ln\left(\frac{I''_{(HI)}}{I''_{(LO)}}\right)}{\lambda_{LO} - \lambda_{HI}}$$

wherein "In" is a natural logarithm.

## Brief Description of the Accompanying Drawings

The embodiments of the disclosure itself, as well as a preferred mode of use, further objectives and advantages thereof, will best be understood by reference to the following detailed description of an illustrative embodiment when read in conjunction with the accompanying drawings. One or more embodiments are now described, by way of example only, with reference to the accompanying drawings in which:

**FIG.1A** shows an exemplary architecture for estimating distance between a fiber end and a target in accordance with some embodiments of the present disclosure;

**FIG.1B** shows an exemplary optical fiber in accordance with some embodiments of the present disclosure;

**FIG.2A-FIG.2F** show exemplary configurations for estimating distance between a fiber end and a target in accordance with some embodiments of the present disclosure;

**FIG.2G** shows an exemplary view of a proximal end of optical fiber, cut at a specific angle, in accordance with some embodiments of the present disclosure;

**FIG.3** illustrates a flowchart showing a method of estimating distance between a fiber end and a target in accordance with some embodiments of the present disclosure;

**FIG.4** is a block diagram of an exemplary computer system for implementing embodiments consistent with the present disclosure.

The figures depict embodiments of the disclosure for purposes of illustration only. One skilled in the art will readily recognize from the following description that alternative embodiments of the structures and methods illustrated herein may be employed without departing from the principles of the disclosure described herein.

## Description of the Disclosure

The foregoing has broadly outlined the features and technical advantages of the present disclosure in order that the detailed description of the disclosure that follows may be better understood. It should be appreciated by those skilled in the art that the conception and specific embodiment disclosed may be readily utilized as a basis for modifying or designing other structures for carrying out the same purposes of the present disclosure. The novel features which are believed to be characteristic of the disclosure, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present disclosure.

**FIG.1A** shows an exemplary architecture for estimating distance between a fiber end and a target in accordance with some embodiments of the present disclosure.

In an embodiment, the exemplary architecture **100** comprises a target **101,** an optical fiber **103,** a Light Emitting, Transmitting and Detecting (LETD) system **105,** a processing unit **107** and an indicator **109.** In some embodiments, the target **101** may be a tissue, a stone, a tumor, a cyst and the like, within a subject, which is required to be treated, ablated or destroyed. In some embodiments, the subject may be a human being or an animal. Further, the optical fiber **103** comprises a proximal end and a distal end. The proximal end is the end of the optical fiber **103** through which light beams enter the optical fiber **103** and the distal end is the end of the optical fiber **103** through which the light beams are directed onto the target **101.** Therefore, the light beams (shown as "incident light" in **FIG. 1B)** enter at the proximal end of the optical fiber **103,** propagate through length of the optical fiber **103** and are directed onto the target **101** from the distal end of the optical fiber **103** as shown in the **FIG.1B**. In some embodiments, the light beams may be beams directed from a light source. As an example, the light source in the context of the present disclosure is a laser light source. As an example, the laser light sources may include, but not limited to, solid state lasers, gas lasers, diode lasers and fiber lasers. The light beams may include, but not limited to, (1) an aiming beam which is a light beam of low intensity and transmitted through the optical fiber 103 to estimate the distance between the optical fiber end and the target 101, but not to treat the target 101, (2) a treatment beam which is a light beam of high intensity and transmitted through the optical fiber 103 to treat the target 101, (3) or any other beam transmitted through the optical fiber 103. In some embodiments, the light beams may be produced by one or more laser light sources. As an example, the aiming beam may be generated by one laser source and the treatment beam may be generated by another laser source. In another example, both the aiming beam and the treatment beam may be generated by a single laser source. In another example, different laser light sources may be used to generate light beams of different wavelengths, characteristics and the like.

Further, the optical fiber **103** may be associated with the LETD system **105** as shown in the **FIG.1A,** to receive the light beams, to be aimed at the target **101,** and to deliver the reflected light beams that reflect from the surface and region around the target **101.** In some embodiments, the optical fiber **103** may be associated with the LETD system **105** via a port (not shown in the **FIG.1A).**

In some embodiments, the LETD system **105** comprises optical components which may include, but not limited to, one or more of laser light sources, polarizers, beam splitters, beam combiners, light detector, wavelength division multiplexers, collimators, circulators, that are configured in various different combinations, as explained in detail in further part of the present disclosure. Functionality of each of the aforementioned optical elements is provided below for a better understanding of the present disclosure.

**Laser light sources:** The laser light sources are configured to generate laser light beams such as low intensity aiming beam for the purpose of aiming the target **101** and high intensity treatment beam for treating the target **101,** and light beams at varying intensities based on the requirement. In some embodiments, each laser light source may be of same wavelength or different wavelengths having different water absorption coefficients. Further, each laser light source may have the same aperture or different apertures. In some embodiments, each laser light source may be designated with a different purpose, for instance, one laser light source may be configured to generate aiming beams of a particular intensity and one laser light source may be configured to generate a treatment beam of a particular intensity, and one or more laser light sources may be configured to generate light beams of a specific wavelength having specific water absorption coefficient.

**Polarizers:** Polarizers are the optical components that act as an optical filter i.e. polarizers are configured to allow light beams of a specific polarization to pass through, and to block the light beams of different polarizations. Therefore, when undefined or light beams of mixed polarity are provided as input to a polarizer, the polarizer provides a well-defined single polarized light beam as an output.

**Beam splitters:** Beam splitters are the optical components used to split incident light at a designated ratio into two separate beams. Further, the beam splitters may be designed to make the light to be incident at a desired Angle of Incidence (AOI), based on the requirement. Therefore, mainly, a beam splitter can be configured with two parameters i.e. a ratio of separation and an AOI. The ratio generally indicates Reflection/Transmission (R/T) ratio. As an example, if the ratio of separation for a beam splitter is indicated as 50:50, it means that the beam splitter splits the incident light beams in a R/T ratio of 50:50 i.e. the beam splitter splits the incident light beams by changing path of 50 percent of the light beams by reflection and other 50 percent by transmission. Further, as an example, if the AOI for the beam splitter is indicated as 45 degrees, it means that the beam splitter ensures that the light beams would be incident at an angle of 45 degrees. Beam splitters may include, but not limited to, polarizing beam splitters and non-polarizing beam splitters. The polarizing beam splitters are designed to split the incident light beams into reflected S-Polarized and transmitted P-polarized beams. The non-polarizing beam splitters are designed to split the incident light beams into specific R/T ratio, but maintain the original polarization state of the incident light beams.

**Beam combiners:** Beam combiners are partial reflectors that combine two or more wavelengths of light, by using the principle of transmission and reflection as explained for a beam splitter. Basically, beam combiner is a combination of beam splitters and mirrors, which perform the functionality of combining light of two or more wavelengths.

**Light detector:** Light detectors are the devices which detect the specific type of light beams (as preconfigured), and converts the light energy associated with the detected light beams into electrical signals.

**Wavelength division multiplexers:** Wavelength division multiplexing is a technology that involves simultaneous transmissions of a number of optical carrier signals onto a single optical fiber while using laser lights of different wavelengths.

**Collimators:** Collimator is a device which narrows down light beams. To narrow down the light beam, the collimator may be configured to cause the directions of motion to become more aligned in a specific direction (for example, parallel rays), or to cause the spatial cross section of the beam to become smaller. In other words, collimator is a device used for changing the diverging light from a point source into a parallel beam.

**Circulators:** Circulators are three or four port optical device designed such that, light entering any one port exits from the next port. These circulators are used to allow light beams to travel in only one direction.

The LETD system **105** is further associated with a processing unit **107** via a communication network. In some embodiments, the communication network may be a wired communication network or a wireless communication network. The processing unit **107** may be configured to receive measured values from the LETD system **105,** to estimate the distance between the distal end of the optical fiber **103** and the target **101.** In some embodiments, the processing unit **107** may be a standalone device with the processing capability required for distance estimation. In some other embodiments, the processing unit **107** may be a computing device such as a laptop, a desktop, a mobile phone, a tablet phone and the like, configured to perform the distance estimation using their processing capability. Further, the processing unit **107** may be associated with the indicator **109** to indicate the estimated distance between the distal end of the optical fiber **103** and the target **101.** As an example, the indicator **109** may include, but not limited to, a visual indicator which displays the estimated distance, an audio indicator which announces the estimated distance, or a haptic indicator which indicates the estimated distance via vibration patterns. In some embodiments, the computing device configured as the processing unit **107** may be configured to perform the functionalities of the indicator **109.** In some other embodiments, the indicator **109** may be a standalone device which is configured to indicate the estimated distance between the distal end of the optical fiber **103** and the target **101.**

In some embodiments, various exemplary configurations for estimating distance between the fiber end and the target are explained in detail below. However, values and parameters associated with different optical components used in each of the below explained configurations, should be considered purely exemplary, and not be construed as a limitation of the present disclosure.

**FIG.2A** shows an exemplary configuration for estimating distance between a fiber end and a target in accordance with some embodiments of the present disclosure.

Exemplary configuration 1:

In this exemplary configuration, the LETD system **105** may include one or more polarized lasers, one or more beam splitters, a polarizer, a beam combiner, and one or more light detectors. The one or more beam splitters may be polarized beam splitters, non-polarized beam splitters or a combination of both polarized and non-polarized beam splitters. As shown in the **FIG.2A,** the LETD system **105** includes a polarized laser source (L1), a polarized laser source (L2), a first beam splitter **203,** a power detector **205,** a polarizer **207,** a first beam combiner **209,** a second beam splitter **211,** a polarized beam splitter **213,** a first light detector **215,** and a second light detector **217.** In this configuration, as shown in the **FIG.2A,** the polarized laser source (L1) has a wavelength with high water absorption coefficient (HI) and the polarized laser source (L2) has a wavelength with low water absorption coefficient (LO). As an example, the laser sources L1 and L2 may be Polarization Maintaining (PM) pigtailed fiber lasers. The incident light beams from L1 and L2 are provided as input to the first beam splitter **203** which is configured to split the incident light beams at a ratio of 50:50, such that the incident light beams of L1 and L2 align along a single optical path. The power detector **205** associated with the first beam splitter **203** may measure the optical power in the optical signal (light beam) corresponding to each wavelength. In some embodiments, the term "optical power" may refer to energy transported by a certain laser beam, per unit time. Further, the output of the first beam splitter **203,** which is the incident light beams aligned along a single optical path, may be provided as an input to a polarizer **207** for providing a well-defined single polarized light beam as an output. In some embodiments, the polarity of the polarizer **207** may be pre-configured. Thereafter, the polarized light obtained as an output from the polarizer **207** may be provided as input to the first beam combiner **209.** The first beam combiner **209** may combine the polarized light beams with an aiming beam and a treatment beam as shown in the **FIG.2A.** In some other embodiments, the aiming beam and the treatment beam may be generated by one or more laser sources other than the laser sources L1 and L2. As an example, the treatment beam may be generated by a solid state laser or a fiber laser such as Holomium (HO) laser. However, this should not be considered as a limitation of the present disclosure, since the treatment beam may be generated by lasers other than Holomium Laser, such as Neodymium, Erbium, Thulium and the like. In some other embodiments, the aiming beam and the treatment beam may be generated by the laser sources L1 and L2. The combined light beam comprising the aiming beam, treatment beam and the polarized light beams from laser sources L1 and L2, may be subjected to the second beam splitter **211** having a configuration of ratio 50:50 and Angle Of Incidence (AOI) 45 degree. The second beam splitter may split the combined light beam in the ratio of 50:50, such that, the aiming beam, treatment beam and the polarized light beams from laser sources L1 and L2, may be aligned along a single optical path. The light beams **221** which is the output of the second beam splitter **211,** are then transmitted to an optical fiber **103** via a port **219** as shown in the **FIG.2A.** The light beams **221** from the second beam splitter **211** are transmitted to the proximal end of the optical fiber **103,** which then propagate through the length of the optical fiber **103** and are delivered to the target **101** from distal end of the optical fiber **103.** As an example, the target **101** may be a tissue, a stone, a tumor, a cyst and the like, within a subject, which is required to be treated, ablated or destroyed. When the light beams **221** are delivered to the target **101** via the optical fiber **103,** the target **101** may reflect some portion of light away from the optical fiber **103** and some portion of the light towards the optical fiber **103,** wherein the portion of light reflected towards the optical fiber **103** may re-enter the optical fiber **103,** at the distal end of the optical fiber **103.** The portion of the reflected light re-entering at the distal end may be referred as reflected light **223a.** The reflected light **223a** may be transmitted backward in optical fiber **103** from the distal end to the proximal end of the optical fiber **103.** When the reflected light **223a** reaches the proximal end of the optical fiber **103,** the reflected light **223a** may be subjected to the second beam splitter **211.** The reflected light **223a** may include numerous reflections from the proximal end of the optical fiber **103,** from the distal end of the optical fiber **103,** and the like, due to which the reflected light **223a** is no longer polarized. In order to polarize the reflected light **223a,** the reflected light is first subjected to the second beam splitter **211** to align the optical path of the reflected light **223a** and then subjected to the polarized beam splitter **213.** The reflected light **223a** would be incident at an angle of 45 degree to the second beam splitter **211** and split in the ratio of 50:50. The reflected light **223b** which emerges out of the second beam splitter **211** is thereafter subjected to the polarized beam splitter **213** as shown in the **FIG.2A.** The polarized beam splitter **213** may split the reflected light **223b** into reflected S-Polarized and transmitted P-polarized beams. In some embodiments, the first light detector **215** may be configured to detect the transmitted P-polarized beams of the reflected light **223b.** In some embodiments, the second light detector **217** may be configured to detect the reflected S-polarized beams of the reflected light **223b.** The first light detector **215** and the second light detector **217** may measure intensities of the detected light beams of the reflected light **223b,** respectively, and transmit the intensities to the processing unit **107.** In some embodiments, the processing unit **107** may estimate distance between the distal end of the optical fiber **103** and the target **101** based on the measured intensities. The method of estimating the distance between the distal end of the optical fiber **103** and the target **101** based on the measured intensities is explained in detail under **FIG.3** in the present disclosure.

**FIG.2B** shows another exemplary configuration for estimating distance between a fiber end and a target in accordance with some embodiments of the present disclosure.

Exemplary configuration 2:

This exemplary configuration is different from the exemplary configuration 1 in two constructional aspects. One of the constructional aspects which is different in this configuration when compared to exemplary configuration 1 is the arrangement of the first beam splitter **203.** In the exemplary configuration 2, the first beam splitter **203** is replaced with a second beam combiner **225.** Since the first beam splitter **203** is replaced with a second beam combiner **225,** the power detector **205** which was associated with the first beam splitter **203** in the exemplary configuration 1, is arranged to be associated with the second beam splitter **211** in the exemplary configuration 2.

In the exemplary configuration 2, the LETD system **105** may include one or more polarized lasers, one or more beam splitters, a polarizer, one or more beam combiners, and one or more light detectors. The one or more beam splitters may be polarized beam splitters, non-polarized beam splitters or a combination of both polarized and non-polarized beam splitters. As shown in the **FIG.2B,** the LETD system **105** includes a polarized laser source (L1), a polarized laser source (L2), a power detector **205,** a polarizer **207,** a first beam combiner **209,** a second beam combiner **225,** a second beam splitter **211,** a polarized beam splitter **213,** a first light detector **215,** and a second light detector **217.** In this configuration, as shown in the **FIG.2B,** the polarized laser source (L1) has a wavelength with high water absorption coefficient (HI) and the polarized laser source (L2) has a wavelength with low water absorption coefficient (LO). As an example, the laser sources L1 and L2 may be Polarization Maintaining (PM) pigtailed fiber lasers. The incident light beams from L1 and L2 are provided as input to the second beam combiner **225** which is configured to combine the incident light beams that are generated by the laser sources L1 and L2. Further, the output of the second beam combiner **225,** may be provided as an input to a polarizer **207** for providing a well-defined single polarized light beam as an output. In some embodiments, the polarization of the polarizer **207** may be pre-configured. Thereafter, the polarized light obtained as an output from the polarizer **207 may** be provided as input to the first beam combiner **209.** The first beam combiner **209** may combine the polarized light beams with an aiming beam and a treatment beam as shown in the **FIG.2B.** In some embodiments, the aiming beam and the treatment beam may be generated by one or more laser sources other than the laser sources L1 and L2. As an example, the treatment beam may be generated by a solid state laser or a fiber laser such as Holmium (HO) laser. However, this should not be considered as a limitation of the present disclosure, since the treatment beam may be generated by lasers other than Holmium Laser, such as Neodymium, Erbium, Thulium and the like. In some other embodiments, the aiming beam and the treatment beam may be generated by the laser sources L1 and L2. The combined light beam comprising the aiming beam, treatment beam and the polarized light beams from laser sources L1 and L2, may be subjected to a second beam splitter **211** having a configuration of ratio 50:50 and Angle Of Incidence (AOI) 45 degree. The second beam splitter may split the combined light beam in the ratio of 50:50, such that, the aiming beam, treatment beam and the polarized light beams from laser sources L1 and L2, may be aligned along a single optical path. The power detector **205** associated with the second beam splitter **211** may measure the power in the optical signal (light beam) corresponding to each wavelength. In some other embodiments, the power detector **205** may detect cumulative energy of the optical signal received at the second beam splitter **211.** In some embodiments, the term "optical power" may refer to energy transported by a certain laser beam, per unit time. The light beams **221** which are the output of the second beam splitter **211,** are then transmitted to an optical fiber **103** via a port **219** as shown in the **FIG.2B.** The light beams **221** from the second beam splitter **211** are transmitted to the proximal end of the optical fiber **103,** which then propagate through the length of the optical fiber **103** and are delivered to the target **101** from distal end of the optical fiber **103.** As an example, the target **101** may be a tissue, a stone, a tumor, a cyst and the like, within a subject, which is required to be treated, ablated or destroyed. When the light beams **221** are delivered to the target **101** via the optical fiber **103,** the target **101** may reflect some portion of light away from the optical fiber **103** and some portion of the light towards the optical fiber **103,** wherein the portion of light reflected towards the optical fiber **103** may re-enter the optical fiber **103,** at the distal end of the optical fiber **103.** The portion of the reflected light re-entering at the distal end may be referred as reflected light **223a.** The reflected light **223a** may be transmitted backward in optical fiber **103** from the distal end to the proximal end of the optical fiber **103.** The reflected light **223a** may include numerous reflections from the proximal end of the optical fiber **103,** from the distal end of the optical fiber **103,** and the like, due to which the reflected light **223a** is no longer polarized. In order to polarize the reflected light **223a,** the reflected light is first subjected to the second beam splitter **211** to align the optical path of the reflected light **223a** and then subjected to the polarized beam splitter **213.** The reflected light **223a** would be incident at an angle of 45 degree to the second beam splitter **211** and split in the ratio of 50:50. The reflected light **223b** which emerges out of the second beam splitter **211** is thereafter subjected to the polarized beam splitter **213** as shown in the **FIG.2B.** The polarized beam splitter **213** may split the reflected light **223b** into reflected S-Polarized and transmitted P-polarized beams. In some embodiments, the first light detector **215** may be configured to detect the transmitted P-polarized beams of the reflected light **223b.** In some embodiments, the second light detector **217** may be configured to detect the reflected S-polarized beams of the reflected light **223b.** The first light detector **215** and the second light detector **217** may measure intensities of the detected light beams of the reflected light **223b,** respectively, and transmit the intensities to the processing unit **107.** In some embodiments, the processing unit **107** may estimate the distance between the distal end of the optical fiber **103** and the target **101** based on the measured intensities. The method of estimating the distance between the distal end of the optical fiber **103** and the target **101** based on the measured intensities is explained in detail in the discussion of **FIG.3** in the present disclosure.

**FIG.2C** shows yet another exemplary configuration for estimating distance between a fiber end and a target in accordance with some embodiments of the present disclosure.

Exemplary configuration 3:

The present disclosure is capable of working with polarized and non-polarized laser sources. Therefore, in this configuration, the laser sources L1' and L2' used for providing incident light beams (source light) are non-polarized laser sources as shown in the **FIG.2C.** As an example, the laser sources L1' and L2' may be Single Mode (SM) fiber pigtailed lasers. When the laser sources L1' and L2' are non-polarized laser sources, there is no requirement of a polarizer **207,** polarized beam splitter **213,** a first light detector **215** for detecting transmitted P-polarized light beams and a second light detector **217** for detecting reflected S-polarized light beams, as required in exemplary configurations 1 and 2.

In the exemplary configuration 3, the LETD system **105** may include one or more non-polarized lasers, one or more beam splitters, a beam combiner, and a light detector. The one or more beam splitters may be non-polarized beam splitters. As shown in the **FIG.2C,** the LETD system **105** includes a non-polarized laser source (L1'), a non-polarized laser source (L2'), a first beam splitter **203,** a power detector **205,** a first beam combiner **209,** a second beam splitter **211,** and a third light detector **227.** In this configuration, as shown in the **FIG.2C,** the non-polarized laser source (L1') has a wavelength with high water absorption coefficient (HI) and the non-polarized laser source (L2') has a wavelength with low water absorption coefficient (LO). The incident light beams from L1' and L2' are provided as input to the first beam splitter **203** which is configured to split the incident light beams at a ratio of 50:50, in a way that, the incident light beams from L1' and L2' align along a single optical path. The power detector **205** associated with the first beam splitter **203** may measure the power in the optical signal (light beam) corresponding to each wavelength. In some embodiments, the term "optical power" may refer to energy transported by a certain laser beam, per unit time. Since, this exemplary configuration 3 is implemented in a non-polarized environment, the optical component "polarizer" and "polarized beam splitter" are eliminated from the configuration. Therefore, the output of the first beam splitter **203,** which is the incident light aligned along a single optical path, may be provided as an input to the first beam combiner **209** that may combine the light beams coming from the first beam splitter **203** with an aiming beam and a treatment beam as shown in the **FIG.2C,** instead of polarizer, as was seen in exemplary configurations 1 and 2. In some embodiments, the aiming beam and the treatment beam may be generated by one or more laser sources other than the laser sources L1' and L2'. As an example, the treatment beam may be generated by a solid state laser or a fiber laser such as Holmium (HO) laser. However, this should not be considered as a limitation of the present disclosure, since the treatment beam may be generated by lasers other than Holmium Laser, such as Neodymium, Erbium, Thulium and the like. In some other embodiments, the aiming beam and the treatment beam may be generated by the laser sources L1' and L2'. The combined light beam comprising the aiming beam, treatment beam and the non-polarized light beams from laser sources L1' and L2', may be subjected to a second beam splitter **211** having a configuration of ratio 50:50 and Angle Of Incidence (AOI) 45 degree. The second beam splitter may split the combined light beam in the ratio of 50:50, such that, the aiming beam, treatment beam and the non-polarized light beams from laser sources L1 and L2, may be aligned along a single optical path. The light beams **221** which are the output of the second beam splitter **211,** are then transmitted to an optical fiber **103** via a port **219** as shown in the **FIG.2C.** The light beams **221** from the second beam splitter **211** are transmitted to the proximal end of the optical fiber **103,** which then propagate through the length of the optical fiber **103** and are delivered to the target **101** from distal end of the optical fiber **103.** As an example, the target **101** may be a tissue, a stone, a tumor, a cyst and the like, within a subject, which is required to be treated, ablated or destroyed. When the light beams **221** are delivered to the target **101** via the optical fiber **103,** the target **101** may reflect some portion of light away from the optical fiber **103** and some portion of the light towards the optical fiber **103,** wherein the portion of light reflected towards the optical fiber **103** may re-enter the optical fiber **103,** at the distal end of the optical fiber **103.** The portion of the reflected light re-entering at the distal end may be referred as reflected light **223a.** The reflected light **223a** may be transmitted backward in optical fiber **103** from the distal end to the proximal end of the optical fiber **103.** The reflected light **223a** may include numerous reflections from the proximal end of the optical fiber **103,** from the distal end of the optical fiber **103,** and the like, due to which the reflected light **223a** is not polarized. Since, this exemplary configuration 3 is implemented in a non-polarized environment, the reflected light **223a** is only subjected to the second beam splitter **211** to align the optical path of the reflected light **223a,** but not to the optical component "polarized beam splitter" as was seen in the exemplary configurations 1 and 2. The reflected light **223a** would be incident at an angle of 45 degrees to the second beam splitter **211** and split in the ratio of 50:50. The reflected light **223b** which emerges out of the second beam splitter **211** may be directly detected by a single detector i.e. the third light detector **227.** The third light detector **227** may measure intensity of the detected light beams of the reflected light **223b,** respectively, and transmit the intensity to the processing unit **107.** In some embodiments, the processing unit **107** may estimate the distance between the distal end of the optical fiber **103** and the target **101** based on the measured intensities. The method of estimating the distance between the distal end of the optical fiber **103** and the target **101** based on the measured intensities is explained in detail in the discussion of **FIG.3** in the present disclosure.

**FIG.2D** shows yet another exemplary configuration for estimating distance between a fiber end and a target in accordance with some embodiments of the present disclosure.

Exemplary configuration 4:

The exemplary configuration 4 is different from the previous exemplary configurations 1-3, in a way that, the exemplary configuration 4 comprises a third polarized laser (L3) which is introduced for the purpose of calibration of the optical fiber condition in real-time. As an example, the condition of the optical fiber **103** may include, but not limited to, any changes or degradation of the distal or proximal ends of the optical fiber **103,** fiber bending effects on polarization scrambling or any other degradations and changes occurring in the optical fiber **103.** Changes in condition of the optical fiber **103,** specifically the tips/ends of the optical fiber **103** may adversely affect the reflected light beams, causing large number of reflections, loss of energy and inaccurate measurements. This may affect the accuracy of the distance estimation, thereby leading to incorrect positioning of the optical fiber **103** during the treatment.

In this exemplary configuration, the LETD system **105** may include one or more polarized lasers, one or more beam splitters, a polarizer, a beam combiner, and one or more light detectors. The one or more beam splitters may be polarized beam splitters, non-polarized beam splitters or a combination of both polarized and non-polarized beam splitters. As shown in the **FIG.2D,** the LETD system **105** includes a polarized laser source (L1), a polarized laser source (L2), a polarized laser source (L3), a first beam splitter **203,** a power detector **205,** a polarizer **207,** a first beam combiner **209,** a second beam splitter **211,** a polarized beam splitter **213,** a first light detector **215,** a second light detector **217,** and a third beam splitter **229.** In this configuration, as shown in the **FIG.2D,** the polarized laser source (L1) has a wavelength with high water absorption coefficient (HI) and the polarized laser source (L2) has a wavelength with low water absorption coefficient (LO). As an example, the laser sources L1 and L2 may be Polarization Maintaining (PM) pigtailed fiber lasers.

The incident light beams from L1 and L2 are provided as input to the first beam splitter **203** which is configured to split the incident light beams at a ratio of 50:50, such that the incident light beams of L1 and L2 align along a single optical path. Further, the output of the first beam splitter **203** which is the incident light beams aligned along a single optical path, may be provided as an input to the third beam splitter **229,** which is also configured to split the light beam in the ratio of 50:50. At the third beam splitter **229,** incident light beams from the polarized laser source (L3) meant for calibration, as shown in the **FIG.2D,** are provided as input along with the output of the first beam splitter **203.** The power detector **205** associated with the third beam splitter **229** may measure the power in the optical signal (light beam) corresponding to each wavelength arriving at the third beam splitter **229.** In some embodiments, the term "optical power" may refer to energy transported by a certain laser beam, per unit time. Along with the output of the first beam splitter **203,** the third beam splitter **229** receives incident light beams from the polarized laser source (L3). In some embodiments, the polarized laser source (L3) has a wavelength with very high water absorption co-efficient, which completely absorbs water. As an example, the wavelength of the polarized laser source (L3) may be 1435nm. Based on the readings of the polarized laser source (L3), fiber feedback mechanism configured in the processing unit **107** may define an optical baseline characteristic of the distal fiber tip quality. Since L3 is highly absorbed in water, it does not reach the target tissue and hardly any L3 back reflection light enters into the fiber as part of the reflected light **223a.** Therefore, L3 reflections **223c** readings mainly reflect the optical characteristics of distal end of the optical fiber. It is the same distal end of the optical fiber which goes through degradation during a laser treatment due to, for example, heat and cavitation. Increased intensities readings of back reflected light **223c,** may indicate optical fiber tip degradation. At a certain threshold of intensity changes from the base line reading for a specific fiber, e.g. 10% - 100%, the controller through a user interface, may indicate that a fiber has to be checked or replaced. In addition, optical fiber tip degradation may cause higher internal reflections from the distal end of the fiber, of polarized laser sources L1 and L2. Moreover, fiber tip degradation may change the ratios between polarities P and S in back reflected light **223a** or **223c.** Therefore, creating base line readings, for a specific fiber currently in use, and monitoring these baselines on the fly, may allow more accurate distance estimations even when and during the tip of the fiber degrades and until degradation reaches the point that a fiber has to be replaced. Further, output of the third beam splitter **229** which includes the incident light beams from L1, L2 and L3 that are aligned along a single optical path, may be provided as an input to the polarizer **207** to obtain a well-defined single polarized light beam as an output. In some embodiments, the polarization of the polarizer **207** may be pre-configured. Thereafter, the polarized light obtained as an output from the polarizer **207** may be provided as input to the first beam combiner **209.** The first beam combiner **209** may combine the polarized light beams with an aiming beam and a treatment beam as shown in the **FIG.2D.** In some embodiments, the aiming beam and the treatment beam may be generated by one or more laser sources other than the laser sources L1 and L2. As an example, the treatment beam may be generated by a solid state laser or a fiber such as Holmium (HO) laser. However, this should not be considered as a limitation of the present disclosure, since the treatment beam may be generated by lasers other than Holmium Laser, such as Neodymium, Erbium, Thulium and the like. In some other embodiments, the aiming beam and the treatment beam may be generated by the laser sources L1 and L2. The combined light beam comprising the aiming beam, treatment beam and the polarized light beams from laser sources L1 and L2, may be subjected to the second beam splitter **211** having a configuration of ratio 50:50 and Angle Of Incidence (AOI) 45 degree. The second beam splitter **211** may split the combined light beam in the ratio of 50:50, such that, the aiming beam, treatment beam and the polarized light beams from laser sources L1 and L2, may be aligned along a single optical path. The light beams **221** which is the output of the second beam splitter **211,** is then transmitted to an optical fiber **103** via a port **219** as shown in the **FIG.2D.** The light beams **221** from the second beam splitter **211** are transmitted to the proximal end of the optical fiber **103,** which then propagate through the length of the optical fiber **103** and are delivered to the target **101** from distal end of the optical fiber **103.** As an example, the target **101** may be a tissue, a stone, a tumor, a cyst and the like, within a subject, which is required to be treated, ablated or destroyed. When the light beams **221** are delivered to the target **101** via the optical fiber **103,** the target **101** may reflect some portion of light away from the optical fiber **103** and some portion of the light towards the optical fiber **103,** wherein the portion of light reflected towards the optical fiber **103** may re-enter the optical fiber **103,** at the distal end of the optical fiber **103.** The portion of the reflected light re-entering at the distal end may be referred as reflected light **223a.** The reflected light **223a** may be transmitted backward in optical fiber **103** from the distal end to the proximal end of the optical fiber **103.** The reflected light **223a** may include numerous reflections from the proximal end of the optical fiber **103,** from the distal end of the optical fiber **103,** and the like, due to which the reflected light **223a** is no longer polarized. In order to polarize the reflected light **223a,** the reflected light is first subjected to the second beam splitter **211** to align the optical path of the reflected light **223a** and then subjected to the polarized beam splitter **213.** The reflected light **223a** would be incident at an angle of 45 degree to the second beam splitter **211** and split in the ratio of 50:50. The reflected light **223b** which emerges out of the second beam splitter **211** is thereafter subjected to the polarized beam splitter **213** as shown in the **FIG.2D.** The polarized beam splitter **213** may split the reflected light **223b** into reflected S-Polarized and transmitted P-polarized beams. In some embodiments, the first light detector **215** may be configured to detect the transmitted P-polarized beams of the reflected light **223b.** In some embodiments, the second light detector **217** may be configured to detect the reflected S-polarized beams of the reflected light **223b.** The first light detector **215** and the second light detector **217** may measure intensities of the detected light beams of the reflected light **223b,** respectively, and transmit the intensities to the processing unit **107.** In some embodiments, the processing unit **107** may estimate distance between the distal end of the optical fiber **103** and the target **101** based on the measured intensities. The method of estimating the distance between the distal end of the optical fiber **103** and the target **101** based on the measured intensities is explained in detail in the discussion of **FIG.3** in the present disclosure.

In some embodiments, the exemplary configuration 4 as discussed above, may be implemented using non-polarized laser sources as well.

**FIG.2E** shows yet another exemplary configuration for estimating distance between a fiber end and a target in accordance with some embodiments of the present disclosure.

Exemplary configuration 5:

This exemplary configuration 5 is firstly implemented in a non-polarized environment. Further, this configuration 5 is a semi-fiber based design, in which, two input beam splitters which were seen in the previous configurations such as exemplary configuration 4, are replaced with a Wavelength Division Multiplexer (WDM). The WDM consumes nearly 50% less energy compared to the beam splitters, which renders it as an efficient system. The exemplary configuration 5 also uses a third non-polarized laser (L3') along with the first and second non-polarized lasers (L1' and L2'). The third non-polarized laser (L3') is introduced for the purpose of calibration of the optical fiber condition in real-time. As an example, the condition of the optical fiber **103** may include, but not limited to, any changes or degradation of the distal or proximal ends of the optical fiber **103,** fiber bending effects on polarization scrambling or any other degradations and changes occurring in the optical fiber **103.** Changes in condition of the optical fiber **103,** specifically tips/ends of the optical fiber **103** may adversely affect the reflected light beams, causing large number of reflections, loss of energy and inaccurate measurements. This may affect the accuracy of the distance estimation, thereby leading to incorrect positioning of the optical fiber **103** during the treatment.

In this exemplary configuration, the LETD system **105** may include one or more non-polarized lasers, one or more beam splitters, a beam combiner, one or more light detectors, a Wavelength Division Multiplexer (WDM), and a collimator. As shown in the **FIG.2E,** the LETD system **105** includes a non-polarized laser source (L1'), a non-polarized laser source (L2'), a non-polarized laser source (L3'), a power detector **205,** a first beam combiner **209,** a second beam splitter **211,** a third light detector **227** and a WDM **231,** a fourth beam splitter **233** and a collimator **235.** In this configuration, as shown in the **FIG.2E,** the non-polarized laser source (L1') has a wavelength with high water absorption coefficient (HI) and the polarized laser source (L2') has a wavelength with low water absorption coefficient (LO). Further, the non-polarized laser source (L3') has a wavelength with very high water absorption co-efficient, which completely absorbs water. As an example, the wavelength of the non-polarized laser source (L3') may be 1435nm. Based on the readings of the non-polarized laser source (L3'), fiber feedback mechanism configured in the processing unit **107** may define an optical baseline characteristic of the distal fiber tip quality. Since L3' is highly absorbed in water, it does not reach the target tissue and hardly any L3' back reflection light enters into the fiber as part of the reflected light **223a.** Therefore, L3' reflections **223c** readings mainly reflect the optical characteristics of distal end of the optical fiber. It is the same distal end of the optical fiber which goes through degradation during a laser treatment due to, for example, heat and cavitation. Increased intensities readings of back reflected light **223c** may indicate optical fiber tip degradation. At a certain threshold of intensity changes from the base line reading for a specific fiber, e.g. 10% - 100%, the controller through a user interface, may indicate that a fiber has to be checked or replaced. In addition, optical fiber tip degradation may cause higher internal reflections from the distal end of the fiber, of non-polarized laser sources L1' and L2'. Moreover, fiber tip degradation may change the ratios between polarities P and S in back reflected light **223a** or **223c.** Therefore, creating base line readings, for a specific fiber currently in use, and monitoring these baselines on the fly, may allow more accurate distance estimations even when and during the tip of the fiber degrades and until degradation reaches the point that a fiber has to be replaced. As an example, the non-polarized laser sources L1', L2' and L3' may be Single Mode (SM) fiber pigtailed lasers.

As mentioned above, in this exemplary configuration 5, the first beam splitter **203** and the third beam splitter **229** as shown in **FIG.2D** are replaced with the WDM **231** as shown in the **FIG.2E.** In some embodiments, to ensure correct usage of the non-polarized laser source (L3'), as a real-time calibrator, it is critical that the incident light beams coming from each of the non-polarized lasers L1', L2' and L3' enter at the proximal end of the optical fiber **103** at the same point and at the same angle. However, such incident light beams from each of the non-polarized lasers L1', L2' and L3' cannot be aligned to enter at the same point and at the same angle, just by using combiners/splitters as was seen in previous exemplary configurations. To ensure adherence with this condition of same point and same angle, the exemplary configuration 5 uses the WDM **231** instead of the beam splitters, at the initial stage, as shown in the **FIG.2E.** The WDM **231** ensures that all the incident light beams coming from each of the non-polarized lasers L1', L2' and L3' enter at the proximal end of the optical fiber **103** at the same point and at the same angle. Moreover, usage of WDM **231** causes lower power loss when compared to the beam splitters which cause 50% - 75% power loss.

The incident light beams from L1', L2', L3' and an aiming beam are provided as inputs to the WDM **231** which is configured to combine the incident light beams in a way that the light beams move identically. Further, output of the WDM **231** may be provided as an input to a fiber based beam splitter i.e. fourth beam splitter **233** that splits the incident light beams at a ratio of 95:5 as shown in the **FIG.2E.** In some embodiments, the fourth beam splitter is a fiber based beam splitter. The power detector **205** associated with the fourth beam splitter **233** may measure the power in the optical signal (light beam) corresponding to each wavelength. In some embodiments, the term "optical power" may refer to energy transported by a certain laser beam, per unit time. Further, the output of the fourth beam splitter **233** which is the incident light aligned along a single optical path, may be provided as an input to a collimator **235** to narrow down the light beams into parallel beams. Thereafter, output of the collimator **235** may be provided to a first beam combiner **209** that combines the light beams coming out of the collimator **235** with an aiming beam and a treatment beam as shown in the **FIG.2E.** In some embodiments, the aiming beam can be either introduced at the beginning into the WDM **231** as shown in the **FIG.2E,** or can be introduced at the first beam combiner **209** as shown in the **FIG.2E,** or can be introduced at both the instances. In some embodiments, the aiming beam and the treatment beam may be generated by one or more laser sources other than the laser sources L1', L2' and L3'. As an example, the treatment beam may be generated by a solid state laser or a fiber laser such as Holmium (HO) laser. However, this should not be considered as a limitation of the present disclosure, since the treatment beam may be generated by lasers other than Holmium Laser, such as Neodymium, Erbium, Thulium and the like. In some other embodiments, the aiming beam and the treatment beam may be generated by the laser sources L1', L2' and L3'. The combined light beam comprising the aiming beam, treatment beam and the light beams from laser sources L1', L2', L3' received from the first beam combiner **209** may be subjected to the second beam splitter **211** having a configuration of ratio 50:50 and Angle Of Incidence (AOI) 45 degrees. The second beam splitter **211** may split the combined light beam in the ratio of 50:50, such that, the aiming beam, treatment beam and the polarized light beams from laser sources L1 and L2, may be aligned along a single optical path. The light beams **221** which are the output of the second beam splitter **211,** are then transmitted to an optical fiber **103** via a port **219** as shown in the **FIG.2E.** The light beams **221** from the second beam splitter **211** are transmitted to the proximal end of the optical fiber **103,** which then propagate through the length of the optical fiber **103** and are delivered to the target **101** from distal end of the optical fiber **103.** As an example, the target **101** may be a tissue, a stone, a tumor, a cyst and the like, within a subject, which is required to be treated, ablated or destroyed. When the light beams **221** are delivered to the target **101** via the optical fiber **103,** the target **101** may reflect some portion of light away from the optical fiber **103** and some portion of the light towards the optical fiber **103,** wherein the portion of light reflected towards the optical fiber **103** may re-enter the optical fiber **103,** at the distal end of the optical fiber **103.** The portion of the reflected light re-entering at the distal end may be referred as reflected light **223a.** The reflected light **223a** may be transmitted backward in optical fiber **103** from the distal end to the proximal end of the optical fiber **103.** When the reflected light **223a** reaches the proximal end of the optical fiber **103,** the reflected light **223a** may be subjected to the second beam splitter **211.** The reflected light **223a** may include numerous reflections from the proximal end of the optical fiber **103,** from the distal end of the optical fiber **103,** and the like, due to which the reflected light **223a** is not polarized. Since, this exemplary configuration 5 is implemented in a non-polarized environment, the reflected light **223a** is only subjected to the second beam splitter **211** to align the optical path of the reflected light **223a,** but not to the optical component "polarized beam splitter" as was seen in the exemplary configurations 1, 2, and 4. The reflected light **223a** is incident at an angle of 45 degree to the second beam splitter **211** and split in the ratio of 50:50. The reflected light **223b** which emerges out of the second beam splitter **211** may be detected by a single detector i.e. the third light detector **227.** The third light detector **227** may measure intensity of the detected light beams of the reflected light **223b,** respectively, and transmit the intensity to the processing unit **107.** In some embodiments, the processing unit **107** may estimate distance between the distal end of the optical fiber **103** and the target **101** based on the measured intensities. The method of estimating the distance between the distal end of the optical fiber **103** and the target **101** based on the measured intensities is explained in detail in the discussion of **FIG.3** in the present disclosure.

**FIG.2F** shows yet another exemplary configuration for estimating distance between a fiber end and a target in accordance with some embodiments of the present disclosure.

Exemplary configuration 6:

This exemplary configuration 6 is also implemented in a non-polarized environment like exemplary configuration 5. Further, this configuration 6 is an all-fiber based design, which does not contain input beam splitters which were seen in the previous configurations such as exemplary configuration 4. The beam splitters are completely replaced by a Wavelength Division Multiplexer (WDM). The WDM consumes nearly 50% less energy compared to the beam splitters, which renders an efficient system. The exemplary configuration 5 also uses a third non-polarized laser (L3') along with the first and second non-polarized lasers (L1' and L2'). The third non-polarized laser (L3') is introduced for the purpose of calibration of the optical fiber condition in real-time. As an example, the condition of the optical fiber 103 may include, but not limited to, any changes or degradation of the distal or proximal ends of the optical fiber **103,** fiber bending effects on polarization scrambling or any other degradations and changes occurring in the optical fiber **103.** Changes in condition of the optical fiber **103,** specifically tips/ends of the optical fiber **103** may adversely affect the reflected light beams, causing large number of reflections, loss of energy and inaccurate measurements. This may affect the accuracy of the distance estimation, thereby leading to wrong movements of the optical fiber **103** during the treatment. Further, an in-line circulator is introduced in this exemplary configuration 6, wherein an in-line circulator is configured to allow the light beams to travel in a single direction.

In this exemplary configuration, the LETD system **105** may include one or more non-polarized lasers, one or more beam splitters, a beam combiner, one or more light detectors, a Wavelength Division Multiplexer (WDM), a circulator and a collimator. As shown in the **FIG.2F,** the LETD system **105** includes a non-polarized laser source (L1'), a non-polarized laser source (L2'), a non-polarized laser source (L3'), a power detector **205,** a first beam combiner **209,** a third light detector **227,** a WDM **231,** a fourth beam splitter **233,** a collimator **235** and a circulator **237.** In this configuration, as shown in the **FIG.2F,** the non-polarized laser source (L1') has a wavelength with high water absorption coefficient (HI) and the polarized laser source (L2') has a wavelength with low water absorption coefficient (LO). Further, the non-polarized laser source (L3') has a wavelength with very high water absorption co-efficient, which completely absorbs water. As an example, the wavelength of the non-polarized laser source (L3') may be 1435nm. Based on the readings of the non-polarized laser source (L3'), fiber feedback mechanism configured in the processing unit **107** may define an optical baseline characteristic of the distal fiber tip quality. Since L3' is highly absorbed in water, it does not reach the target tissue and hardly any L3' back reflection light enters into the fiber as part of the reflected light **223a.** Therefore, L3' reflections **223c** readings mainly reflect the optical characteristics of distal end of the optical fiber. It is the same distal end of the optical fiber which goes through degradation during a laser treatment due to, for example, heat and cavitation. Increased intensities readings of back reflected light **223c,** may indicate optical fiber tip degradation. At a certain threshold of intensity changes from the base line reading for a specific fiber, e.g. 10% - 100%, the controller through a user interface, may indicate that a fiber has to be checked or replaced. In addition, optical fiber tip degradation may cause higher internal reflections from the distal end of the fiber, of non-polarized laser sources L1' and L2'. Moreover, fiber tip degradation may change the ratios between polarities P and S in back reflected light **223a** or **223c.** Therefore, creating base line readings, for a specific fiber currently in use, and monitoring these baselines on the fly, may allow more accurate distance estimations even when and during the tip of the fiber degrades and until degradation reaches the point that a fiber has to be replaced. As an example, the non-polarized laser sources L1', L2' and L3' may be Single Mode (SM) fiber pigtailed lasers.

As mentioned above, in this exemplary configuration 6, the first beam splitter **203** and the third beam splitter **229** as shown in **FIG.2D** are replaced with the WDM **231** as shown in the **FIG.2F.** Further, in this exemplary configuration 6, the second beam splitter **211** which was arranged to deliver the light beams to the port **219** in all the aforementioned exemplary configurations, is also eliminated, thereby making this exemplary configuration 6, an all-fiber design. Beam splitter reduces output power by 50% and reduces additional 50% of output power upon receiving return signal. Therefore, removal of the beam splitter in this exemplary configuration 6 significantly increases the signal and the output power.

In some embodiments, to ensure correct usage of the non-polarized laser source (L3'), as a real-time calibrator, it is critical that the incident light beams coming from each of the non-polarized lasers L1', L2' and L3' enter at the proximal end of the optical fiber **103** at the same point and at the same angle. However, such incident light beams from each of the non-polarized lasers L1', L2' and L3' cannot be aligned to enter at the same point and at the same angle, just by using combiners/splitters as was seen in previous exemplary configurations. To ensure adherence with this condition of same point and same angle, the exemplary configuration 6 uses the WDM **231** instead of the beam splitters, at the initial stage, as shown in the **FIG.2F.** The WDM **231** ensures that all the incident light beams coming from each of the non-polarized lasers L1', L2' and L3' enter at the proximal end of the optical fiber **103** at the same point and at the same angle. Moreover, usage of WDM **231** causes lower power loss when compared to the beam splitters which cause 50% - 75% power loss.

The incident light beams from L1', L2', L3' and an aiming beam are provided as inputs to the WDM **231** which is configured to combine the incident light beams in a way that the light beams move identically. Further, output of the WDM **231** may be provided as an input to the fourth beam splitter **233** that splits the incident light beams at a ratio of 95:5 as shown in the **FIG.2F.** In some embodiments, the fourth beam splitter **233** is a fiber based beam splitter. The power detector **205** associated with the fourth beam splitter **233** may measure the power in the optical signal (light beam) corresponding to each wavelength. In some embodiments, the term "optical power" may refer to energy transported by a certain laser beam, per unit time. Further, the output of the fourth beam splitter **233** which is the incident light aligned along a single optical path, may be provided as an input to a circulator **237.** The circulator **237** ensures that all the light beams are allowed to travel in one direction, and provides the light beams to the collimator **235,** from a port other than the port into which the light beams entered, in order to narrow down the light beams into parallel beams. Thereafter, output of the collimator **235** may be provided to a first beam combiner **209** that combines the light beams coming out of the collimator **235** with an aiming beam and a treatment beam as shown in the **FIG.2F.** In some embodiments, the aiming beam can be either introduced at the beginning into the WDM **231** as shown in the **FIG.2F,** or can be introduced at the first beam combiner **209** as shown in the **FIG.2F,** or can be introduced at both the instances. In some embodiments, the aiming beam and the treatment beam may be generated by one or more laser sources other than the laser sources L1', L2' and L3'. As an example, the treatment beam may be generated by a solid state laser or a fiber laser such as Holmium (HO) laser. However, this should not be considered as a limitation of the present disclosure, since the treatment beam may be generated by lasers other than Holmium Laser, such as Neodymium, Erbium, Thulium and the like. In some embodiments, the aiming beam and the treatment beam may be generated by the laser sources L1' and L2'. The combined light beam comprising the aiming beam, treatment beam and the light beams from laser sources L1', L2', L3' received from the first beam combiner **209** may be transmitted to an optical fiber **103** via a port **219** as shown in the **FIG.2F.** The light beams **221** are transmitted to the proximal end of the optical fiber **103,** which then propagate through the length of the optical fiber **103** and are delivered to the target **101** from distal end of the optical fiber **103.** As an example, the target **101** may be a tissue, a stone, a tumor, a cyst and the like, within a subject, which is required to be treated, ablated or destroyed. When the light beams **221** are delivered to the target **101** via the optical fiber **103,** the target **101** may reflect some portion of light away from the optical fiber **103** and some portion of the light towards the optical fiber **103,** wherein the portion of light reflected towards the optical fiber **103** may re-enter the optical fiber **103,** at the distal end of the optical fiber **103.** The portion of the reflected light re-entering at the distal end may be referred as reflected light **223a.** The reflected light **223a** may be transmitted backward in optical fiber **103** from the distal end to the proximal end of the optical fiber **103.** When the reflected light **223a** reaches the proximal end of the optical fiber **103,** the reflected light **223a** may be subjected to the circulator **237.** The reflected light **223a** may include numerous reflections from the proximal end of the optical fiber **103,** from the distal end of the optical fiber **103,** and the like, due to which the reflected light **223a** is not polarized. Since, this exemplary configuration 6 is implemented in a non-polarized environment, the reflected light **223a** is subjected to the circulator **237,** but not to the optical component "polarized beam splitter" as was seen in the exemplary configurations 1, 2, and 4. The reflected light **223b** which emerges out of the circulator **237,** in a single direction, may be detected by a single detector i.e. the third light detector **227.** The third light detector **227** may measure intensity of the detected light beams of the reflected light **223b,** respectively, and transmit the intensity to the processing unit **107.** In some embodiments, the processing unit **107** may estimate distance between the distal end of the optical fiber **103** and the target **101** based on measured intensities. The method of estimating the distance between the distal end of the optical fiber **103** and the target **101** based on measured intensities is explained in detail in the discussion of **FIG.3** in the present disclosure.

In some embodiments, in each of the aforementioned exemplary configurations, the proximal end of optical fiber **103** may be coated with a special coating such as an Anti Reflective (AR) coating. The AR coating helps in reducing the noise created at the proximal end of the optical fiber **103** and increase the dynamic range. In some embodiments, the light signal (reflected light beam) that enters the light detector may contain:

a. Reflections from Port Lens
b. Reflections from Blast Shield
c. Reflections from the proximal end of the optical fiber
d. Reflections from the distal end of the optical fiber

AR coating at the proximal end of the optical fiber **103** may reduce (a) reflections from the port lens to less than 1%, (b) reflections from the blast shield to less than 1%, (c) reflections from the proximal end of the optical fiber **103** to nearly 3.5%, and (d) reflections from the distal end of the optical fiber **103** to nearly 0.2%. In some embodiments, the reflected signal from a target **101** such as stone, may be of very low energy, for instance nearly 1% of fiber output power where the distance from the optical fiber tip to the tissue is about 0mm. By reducing the reflections from the proximal end of the optical fiber **103** to nearly 0.5%, the present disclosure may help in improving the dynamic range of the signals reflected from the target **101.**

In some other embodiments, in each of the aforementioned exemplary configurations, the Sub-Miniature version A (SMA) connector, which is basically at the proximal end of optical fiber **103** may be polished or cut at an angle of 8 degrees, as shown in the **FIG.2G.** Cutting in a slant fashion at an 8 degree angle, as shown in the **FIG.2G,** achieves diversion of the reflected light beams (unwanted reflections caused from proximal end, distal end and the like) from the proximal end of the optical fiber **103,** which in turn may reduce substantive noise and increase dynamic range. In some embodiments, the light signal (reflected light beam) that enters the light detector may contain:

a. Reflections from Port Lens
b. Reflections from Blast Shield
c. Reflections from the fiber Proximal end
d. Reflections from fiber Distal end

As explained above, AR coating at the proximal end of the optical fiber **103** may reduce (a) reflections from the port lens to less than 1%, (b) reflections from the blast shield to less than 1%, (c) reflections from the proximal end of the optical fiber **103** to nearly 3.5%, and (d) reflections from the distal end of the optical fiber **103** to nearly 0.2%. However, the angled finer proximal end of the optical fiber **103** helps in reducing unwanted reflections, and improves the dynamic range of the signals reflected from the target **101.**

**FIG.3** illustrates a flowchart showing a method of estimating distance between a fiber end and a target in accordance with some embodiments of the present disclosure.

**At block 301,** the method includes illuminating, by a Light Emitting, Transmitting and Detecting (LETD) system **105,** a target **101** with laser light of plurality of different wavelengths, via the optical fiber **103,** using plurality of laser light sources. In some embodiments, the laser light of the plurality of different wavelengths may be provided to the optical fiber **103** for illuminating the target **101** using one of the exemplary configurations 1-6 discussed above in the present disclosure. In some embodiments, the present disclosure may use two wavelengths with different water absorption coefficients in order to ensure robustness with respect to different type of targets **101,** target compositions, target colors, target surfaces and the like. In some embodiments, the two wavelengths may be selected such that, one is a wavelength with low water absorption coefficient (LO) and another is a wavelength with high water absorption coefficient (HI). As an example, the two wavelengths may be 1310 nm and 1340 nm. However, this example should not be construed as a limitation, as different wavelengths with different water absorption coefficients can be used. In some embodiments, two laser sources such as L1 and L2 may be used to emit light of two different wavelengths as mentioned above. In some embodiments, the laser light sources may be at least one of polarized laser sources, non-polarized laser sources or a combination of polarized and non-polarized laser sources. As an example, to measure the distance between the end of the optical fiber **103** and the target **101,** a low-power InfraRed (IR) laser may be used, without limitation, to illuminate the target **101** via the optical fiber **103.**

**At block 303,** the method includes receiving, by the LETD system **105,** reflected light beams from the target **101,** via the optical fiber **103.** In some embodiments, the reflected light beams may include a mixture of reflections from distal end of the optical fiber **103,** from proximal end of the optical fiber **103,** from blast shield and the like. The LETD system **105** may be configured to identify the reflected light beams suitable for measuring intensity.

**At block 305,** the method includes measuring, by the LETD system **105,** intensity of reflected light beams (also known as returned signal) by detecting the returned signal using the one or more light detectors configured in LETD system **105.** In some embodiments, since two different wavelengths are used for illuminating the target **101,** the measured intensities are with respect to two different wavelengths. Therefore, the two measured intensities corresponding to two different wavelengths of the laser sources may be transmitted to a processing unit **107** associated with the LETD system **105.**

At **block 307,** the method includes receiving, by the processing unit **107,** the measured intensities of the returned signal transmitted from the LETD system **105.**

At **block 309,** the method includes estimating, by the processing unit **107,** distance between the distal end of the optical fiber **103** and the target **101** based on the measured intensities of the returned signal. In some embodiments, the processing unit **107** may substitute the measured intensities in the **Equation 1** as shown below:

$$\text{Intensity of the returned signal} = \boldsymbol{R} * \boldsymbol{e}^{(-\boldsymbol{\lambda} * \boldsymbol{X})} \text{ ---------- } \textbf{Equation 1}$$

In the above **Equation 1,**

"R" refers to target reflection coefficient which is affected by target composition, target color/pigment, target angle, target surface and the like;
"$\lambda$" refers to water absorption coefficient of a specific wavelength; and
"X" refers to distance between the distal end of the optical fiber 103 and the target **101.**

In the above **Equation 1,** "X" and "R" are unknown parameters which need to be determined by the processing unit **107.** Therefore, in order to determine the values of "X" and "R", the processing unit **107** may substitute the two measured intensity values in the above **Equation 1,** thereby obtaining two equations with substituted values of measured intensity and the water absorption coefficient of the corresponding wavelength. For instance, the two equations with substituted values may be as shown below.

$$\mathbf{I}_{(\mathbf{HI})} = \boldsymbol{R} * \boldsymbol{e}^{(-\boldsymbol{\lambda_HI} * \boldsymbol{X})} \text{ ----- } \textbf{Equation 1.1}$$

$$\mathbf{I}_{(\mathbf{LO})} = \boldsymbol{R} * \boldsymbol{e}^{(-\boldsymbol{\lambda_LO} * \boldsymbol{X})} \text{ ----- } \textbf{Equation 1.2}$$

The processing unit **107** may further simplify the above mentioned substituted **Equations 1.1 and 1.2** as shown in the below two steps:

Step 1: Compute ratio of measured intensity values obtained for the returned signal of two different wavelengths.

$$\frac{I_{(HI)}}{I_{(LO)}} = \frac{R}{R} * e^{(\lambda_{LO}-\lambda_{HI})*X} \text{ --------- Equation 2.1}$$

Step 2: Determine distance value using the natural logarithm as shown below:

$$X = \frac{\ln(\frac{I_{(HI)}}{I_{(LO)}})}{\lambda_{LO}-\lambda_{HI}} \text{ --------- Equation 2.2}$$

Therefore, the processing unit **107** may estimate the distance (X) between the distal end of the optical fiber **103** and the target **101,** by simplifying the equations 1.1 and 1.2 as shown above. In the above **Equation 2.2,** "In" refers to natural logarithm. In some embodiments, the distance (X) may be measured in millimeters. Upon determining the value of "X", the processing unit **107** may use this value in the **Equation 1.1 and 1.2** to determine the value of "R". In some embodiments, "X" is the same distance for both wavelengths and R (target reflection) is almost identical for both wavelengths if the selected wavelengths are close to each other on the "nm scale" [e.g. 1310nm and 1340nm]. Therefore, selection of wavelengths of low and high absorption coefficients such that the wavelengths are close to each other on the "nm scale" is of utmost importance in distance estimation.

In some embodiments, condition of the optical fiber **103** may be affected due to factors such as changes or degradation of the distal or proximal ends of the optical fiber **103,** fiber bending effects on polarization scrambling or any other degradations and changes occurring in the optical fiber **103.** Changes in optical conditions of the optical fiber **103,** specifically the tips/ends of the optical fiber **103,** may adversely affect the quality of the irradiated beam, the intensity of the internal reflected light beams, the amount of back reflected light from a target which enters the fiber, loss of energy reaches a target and inaccurate measurements. This may affect the accuracy of the distance estimation, thereby leading to incorrect positioning of the optical fiber **103** during the treatment or miscalculating energy optimization which are based on distance estimation as described in the applicant's US provisional patent application no. 63/118,117 which is incorporated herein by reference.

Internal reflections from planes associated with the fiber e.g. the fiber proximal end or the fiber distal end, or that associated with other optical elements which are optically connected with the fiber e.g. lenses or shields, generate parasitic and unwanted reflections. Moreover, these internal reflections may change over time due to fiber or other elements degradation. In addition, fiber degradation may change over time the quality of the laser beam irradiated toward the a target tissue and may also change the intensity of back reflected light from a target tissue which enters and passes the fiber as beams 223a and 223b. To keep accurate distance measurements during fiber degradation and changes in internal reflections, according to an embodiment, there is a need to measure the internal reflections of each laser before a treatment starts, register these values and monitor their changes over time. This process may done for any single fiber is being used with the laser system. Distance measurements, as will be explained below, may be corrected by monitoring these changes. Processing unit **107** is configured to read baseline values of such parasitic/unwanted reflections by a system pre-treatment calibration process. In some embodiments, the system pre-treatment calibration process may include setting up a treatment fiber in water with no target. In this context, "no target" for instance could mean that, the closest target such as stone may be located further away from the tip of the fiber so that no reflections from the target tissue may back reflect into the fiber as signal 223a. Such a distance is for example 10mm from the distal end of the optical fiber **103** or more. Thereafter, under these conditions, the system may shoot the lasers such as (L1 and L2) in a polarized environment, or laser (L1' and L2') in a non-polarized environment. Since the back reflected light 223a under these conditions is very low, the signals reaching the light detectors are related mainly to internal reflections associated with the optical fiber. The Internal Reflected (IR) light beams in such a scenario may be detected using the light detectors and the measured intensity values may be stored as $IR_{(HI)}$ and $IR_{(LO)}$, by the processing unit **107.** $IR_{(HI)}$ is the intensity of the internal reflections of incident light having higher water absorption co-efficient when there is no target close to the fiber tip and $IR_{(LO)}$ is the intensity of internal reflections of incident light having low water absorption co-efficient when there is no target close to the fiber tip. Thereafter, during the normal work, when the target is placed at a distance close enough to the distal end of the optical fiber **103** and may generate signal 223a, the reflected light beams from the target may be detected using the light detectors and the measured intensity values may be stored as $I_{(HI)}$ which is the "Intensity of returned signal from a target tissue corresponding to wavelength having higher water absorption co-efficient (HI)" and $I_{(LO)}$ which is the "Intensity of returned signal from a target tissue corresponding to wavelength having lower water absorption co-efficient (LO)" by the processing unit **107.** However, in order to eliminate values of the parasitic/unwanted reflections from readings of the actual returned signals, the processing unit **107** may subtract/reduce the $IR_{(HI)}$ from reading of the actual returned signal $I_{(HI)}$ as shown in the below **Equation 3.1,** and $IR_{(LO)}$ from reading of the actual returned signal $I_{(LO)}$ as shown in the below **Equations 3.1** and **3.2,** respectively.

$$I'_{(HI)} = I_{(HI)} - IR_{(HI)} \text{ -------------- Equation 3.1}$$

$$I'_{(LO)} = I_{(LO)} - IR_{(LO)} \text{ -------------------- Equation 3.2}$$

In the above **Equation 3.1,**

**I'**$_{(HI)}$ refers to a new calculated Intensity of returned signal corresponding to wavelength having higher water absorption co-efficient (HI) (without the parasitic/unwanted reflections);
**I**$_{(HI)}$ refers to a measured Intensity of returned signal corresponding to wavelength having higher water absorption co-efficient (HI) (with the parasitic/unwanted reflections); and
***IR***$_{(HI)}$ refers to a measured intensity of internal reflections of incident light having higher water absorption co-efficient (measured with "no target")

In the above **Equation 3.2,**
**I'**$_{(LO)}$ refers to new a calculated Intensity of returned signal corresponding to wavelength having lower water absorption co-efficient (LO) (without the parasitic/unwanted reflections);
**I**$_{(LO)}$ refers to a measured Intensity of returned signal corresponding to wavelength having lower water absorption co-efficient (LO) (with the parasitic/unwanted reflections); and
**IR**$_{(LO)}$ refers to measured intensity of internal reflections of incident light having lower water absorption co-efficient (measured with "no target").

Therefore, using the new intensity calculated values **I'**$_{(HI)}$ and **I'**$_{(LO)}$, the processing unit **107** may determine the distance between distal end of the optical fiber **103** and the target **101,** by substituting the new calibrated values **I'**$_{(HI)}$ and **I'**$_{(LO)}$, in **Equation 2.2** as shown below:

$$X = \frac{\ln\left(\frac{I_{(HI)} - IR_{(HI)}}{I_{(LO)} - IR_{(LO)}}\right)}{\lambda_{LO} - \lambda_{HI}}$$

In some embodiments, the above equation of "X" may also be indicated as shown below:

$$X = \frac{\ln\left(\frac{I'_{(HI)}}{I'_{(LO)}}\right)}{\lambda_{LO} - \lambda_{HI}}$$

As mentioned above, the internal reflections may not be constant over time and may change due to some changes in internal optical parameters of the system (as opposed to changes due to the dynamic of the treatment environment which is external to the system) such as the optical quality of the distal end of the optical fiber **103.** Due to the high power of the treating laser and some cavitational effects which take place at the tip of the fiber, due to the lilquid environment of the treatment, the fiber and mainly the distal tip of the fiber, undergoes degradation. Therefore, for monitoring and rectifying such changes in internal reflections in real time, performing real-time calibration is important. Therefore, for performing real-time calibration, as shown in the embodiments 4, 5 and 6 in the present disclosure above, a calibration laser either polarized (L3) or non-polarized (L3') is introduced, thereby ensuring more accurate distance estimation during degradation of the optical fiber. As explained in the embodiments 4, 5 and 6, the calibration laser (L3/L3') has a wavelength with a very high absorption co-efficient in water. As an example, the wavelength of the polarized laser source (L3) may be 1435nm. Since calibration laser (L3/L3') is so strongly absorbed by the liquid environment, as explained above, hardly any back reflection 223a from the media goes back into the fiber. Therefore, while shooting calibration laser (L3/L3'), the associated light detectors mainly measure the intensity of the internal reflections of laser (L3/L3'). Processing unit 107 is configured to read and store one or more base values for the internal reflections of laser (L3/L3') before a treatment starts. These one or more base values represent the optical quality of the fiber, such as the optical quality of the tip of the fiber, before the treatment starts. Further, processing unit 107 is configured to continue measuring on real time and during a treatment, internal reflections of calibration laser (L3/L3') and to identify deviations from the base. Monitoring these deviations provide an indication to a degradation of the optical quality of the fiber and may be used to correct any measured back reflected intensity associated with signal 223a. Based on the readings of the internal reflections by calibration laser (L3/L3'), processing unit **107** may rectify calibration parameters for the main lasers (L1/L1') and (L2/L2') measurements.

In some embodiments, the real-time calibration process may include, initially setting up a treatment fiber in water and read and store one or more internal reflections values of calibration laser (L3/L3'). Since calibration laser (L3/L3') is so highly absorbed in water, there is a much less sensitivity, relative to lasers L1/L1' and L2/L2', to the distance to a target tissue during the calibration readings of L3/L3' . As will explained below, this allow the continuation of calibration laser measurements during treatment when a target tissue may also be close to the tip of the fiber. Thereafter, the target may be illuminated using one of the exemplary configurations 4, 5 or 6 as discussed above in the present disclosure, using lasers (L1, L2) in a polarized environment, or laser (L1', L2') in a non-polarized environment. The reflected light beams 223a and 223b in such a scenario may be detected using the light detectors and the measured intensity values may be stored as $I_{(HI)}$, $I_{(LO)}$ together with additional and associated measurements of the internal reflections of calibration laser (L3/L3') $IR_{(CAL)}$, by the processing unit **107.** $I_{(HI)}$ is the intensity of the back reflections from the target tissue of incident light having higher water absorption coefficient, $I_{(LO)}$ is the intensity of the back reflections from the target tissue of incident light having low water absorption co-efficient, and $IR_{(CAL)}$ is the intensity of the internal reflections of incident calibration laser (L3/L3'). In some embodiments, presence or absence of target may not affect the reflections $IR_{(CAL)}$, since the incident light from the calibration sensors (L3, L3') is highly absorbed by water,. In some embodiments, changes in the $IR_{(CAL)}$ value may occur due to changes in degradation of the optical fiber **103,** specifically tips of the optical fiber **103.** In some embodiments, based on relative changes of the $IR_{(CAL)}$ value, the processing unit **107** may adjust the previously measured $IR_{(HI)}$ and $IR_{(LO)}$ values or the currently measured $I_{(LO)}$ or $I_{(HI)}$.

Thereafter, during the real-time working i.e. when the real-time treatment is going on, when there is presence of target **101** i.e. when the target **101** is at a distance close enough to generate back reflection signal 223a, such as when the target is in a distance lesser than 10mm from the distal end of the optical fiber **103,** the back reflected light beams 223a for laser L1/L1' and for laser L2/L2' and the internal reflection 223c from calibration laser L3/L3', may be detected using the light detectors and the measured intensity values may be stored as $I_{(HI)}$ which is the "Intensity of returned signal corresponding to wavelength having higher water absorption co-efficient (HI)", $I_{(LO)}$ which is the "Intensity of returned signal corresponding to wavelength having lower water absorption co-efficient (LO)" by the processing unit **107,** and $IR_{(CAL)}$ which is the "Intensity of returned internal reflection signal corresponding to wavelength having highest water absorption co-efficient of the calibration laser (CAL)" by the processing unit **107.** Further, in order to determine a calibration factor, the processing unit **107** may divide reading of $IR_{(CAL\text{-}PRE)}$ from the calibration process pre-treatment from reading of $IR_{(CAL\text{-}DUR)}$ from a calibration process done during a treatment as shown in the below **Equation 4.**

$$\text{Calibration Factor (CF)} = \frac{IR_{(CAL-PRE)}}{IR_{(CAL-DUR)}} \text{ -------------- } \textbf{Equation 4}$$

As long as the internal reflections of calibration laser L3/L3' before an during a treatment are the same, when there are no changes in the optical fiber **103,** the calibration factor may be "1". Further, in order to calibrate the rectify parameters for the main lasers (L1/L1') and (L2/L2'), the processing unit **107** may use the calibration factor as shown in the below **Equations 5.1** and **5.2.**

$$\mathbf{I''_{(HI)} = I_{(HI)} - IR_{(HI)} \times CF} \text{ -------------- } \textbf{Equation 5.1}$$

$$\mathbf{I''_{(LO)} = I_{(LO)} - IR_{(LO)} \times CF} \text{ -------------------- } \textbf{Equation 5.2}$$

In the above **Equation 5.1,**

- $\mathbf{I''_{(HI)}}$ refers to a new calculate calibrated Intensity of back reflected signal from a target tissue which is corresponding to a wavelength having higher water absorption co-efficient (HI);
- $\mathbf{I_{(HI)}}$ refers to the measured Intensity of the back reflected signal from a target tissue which is corresponding to a wavelength having higher water absorption co-efficient **(HI);**
- $\mathbf{IR}_{(HI)}$ refers to the measured intensity of the internal reflection of incident laser having higher water absorption co-efficient (measured with "no target"); and
- **CF** refers to calibration factor determined using **Equation 4.**

In the above **Equation 5.2,**

**I"$_{(LO)}$** refers to a new calculate calibrated Intensity of back reflected signal from a target tissue which is corresponding to wavelength having lower water absorption co-efficient (LO);

**I$_{(LO)}$** refers to the measured Intensity of back reflected signal from a target tissue which is corresponding to wavelength having lower water absorption coefficient (LO);

**IR$_{(LO)}$** refers to the measured intensity of internal reflection of incident laser having lower water absorption co-efficient (measured with "no target"); and

**CF** refers to calibration factor determined using **Equation 4.**

Therefore, using the new calibrated intensity values **I"$_{(HI)}$** and **I"$_{(LO)}$**, the processing unit **107** may determine the distance between distal end of the optical fiber **103** and the target **101,** by substituting the new calibrated values **I"$_{(HI)}$** and **I"$_{(LO)}$**, in **Equation 2.2** as shown below:

$$X = \frac{\ln\left(\frac{I_{(HI)} - IR_{(HI)} * CF}{I_{(LO)} - IR_{(LO)} * CF}\right)}{\lambda_{LO} - \lambda_{HI}}$$

Therefore, in this way, the processing unit **107** performs the system pre-treatment calibration and real-time calibration, to ensure accuracy of the estimated distance between the distal end of the optical fiber **103** and the target **101** when the fiber undergoes degradation, and the calibration factor is changed.

At **block 311,** the method includes indicating, by the processing unit **107,** the estimated distance between the distal end of the optical fiber **103** and the target **101,** via an indicator **109** associated with the processing unit **107.** As an example, the indicator **109** may be a visual indicator, an audio indicator or a haptic indicator. In some embodiments, based on the estimated distance between the distal end of the optical fiber **103** and the target **101,** the position of the optical fiber **103** may be varied, the orientation of the optical fiber **103** may be varied, characteristics of the treatment beam may be varied, and the like, in real-time, in order to accurately aim at the target **101.**

**FIG.4** is a block diagram of an exemplary computer system for implementing embodiments consistent with the present disclosure.

In some embodiments, **FIG.4** illustrates a block diagram of an exemplary computer system **400** for implementing embodiments consistent with the present invention. In some embodiments, the computer system **400** can be a processing unit **107** to estimate distance between end of an optical fiber **103** and a target **101.** The computer system **400** may include a central processing unit ("CPU" or "processor") **402.** The processor **402** may include at least one data processor for executing program components for executing user or system-generated business processes. A user may include a person, a person using a device such as those included in this invention, or such a device itself. The processor **402** may include specialized processing units such as integrated system (bus) controllers, memory management control units, floating point units, graphics processing units, digital signal processing units, etc.

The processor **402** may be disposed in communication with input devices **411** and output devices **412** via I/O interface **401.** The I/O interface **401** may employ communication protocols/methods such as, without limitation, audio, analog, digital, stereo, IEEE-1394, serial bus, Universal Serial Bus (USB), infrared, PS/2, BNC, coaxial, component, composite, Digital Visual Interface (DVI), high-definition multimedia interface (HDMI), Radio Frequency (RF) antennas, S-Video, Video Graphics Array (VGA), IEEE 802.n /b/g/n/x, Bluetooth, cellular (e.g., Code-Division Multiple Access (CDMA), High-Speed Packet Access (HSPA+), Global System For Mobile Communications (GSM), Long-Term Evolution (LTE), WiMax, or the like), etc.

Using the I/O interface **401,** computer system **400** may communicate with input devices **411** and output devices **412.**

In some embodiments, the processor **402** may be disposed in communication with a communication network **409** via a network interface **403.** The network interface **403** may communicate with the communication network **409.** The network interface **403** may employ connection protocols including, without limitation, direct connect, Ethernet (e.g., twisted pair 10/100/1000 Base T), Transmission Control Protocol/Internet Protocol (TCP/IP), token ring, IEEE 802.11a/b/g/n/x, etc. Using the network interface **403** and the communication network **409,** the computer system **400** may communicate with Light Emitting, Transmitting and Detecting (LETD) system **105** and an indicator **109.** The communication network **409** can be implemented as one of the different types of networks, such as intranet or Local Area Network (LAN), Closed Area Network (CAN) and such. The communication network **409** may either be a dedicated network or a shared network, which represents an association of the different types of networks that use a variety of protocols, for example, Hypertext Transfer Protocol (HTTP), CAN Protocol, Transmission Control Protocol/Internet Protocol (TCP/IP), Wireless Application Protocol (WAP), etc., to communicate with each other. Further, the communication network **409** may include a variety of network devices, including routers, bridges, servers, computing devices, storage devices, etc. In some embodiments, the processor **402** may be disposed in communication with a memory **405** (e.g., RAM, ROM, etc. not shown in **FIG.4)** via a storage interface **404.** The storage interface **404** may connect to memory **405** including, without limitation, memory drives, removable disc drives, etc., employing connection protocols such as Serial Advanced Technology Attachment (SATA), Integrated Drive Electronics (IDE), IEEE-1394, Universal Serial Bus (USB), fiber channel, Small Computer Systems Interface (SCSI), etc. The memory drives may further include a drum, magnetic disc drive, magnetooptical drive, optical drive, Redundant Array of Independent Discs (RAID), solid-state memory devices, solid-state drives, etc.

The memory **405** may store a collection of program or database components, including, without limitation, a user interface **406,** an operating system **407,** a web browser **408** etc. In some embodiments, the computer system **400** may store user/application data, such as the data, variables, records, etc. as described in this invention. Such databases may be implemented as fault-tolerant, relational, scalable, secure databases such as Oracle or Sybase.

The operating system **407** may facilitate resource management and operation of the computer system **400.** Examples of operating systems include, without limitation, APPLE® MACINTOSH® OS X®, UNIX®, UNIX-like system distributions (E.G., BERKELEY SOFTWARE DISTRIBUTION® (BSD), FREEBSD®, NETBSD®, OPENBSD, etc.), LINUX® DISTRIBUTIONS (E.G., RED HAT®, UBUNTU®, KUBUNTU®, etc.), IBM®OS/2®, MICROSOFT® WINDOWS® (XP®, VISTA®/7/8, 10 etc.), APPLE® IOS®, GOOGLE™ ANDROID™, BLACKBERRY® OS, or the like. The User interface **406** may facilitate display, execution, interaction, manipulation, or operation of program components through textual or graphical facilities. For example, user interfaces may provide computer interaction interface elements on a display system operatively connected to the computer system **400,** such as cursors, icons, checkboxes, menus, scrollers, windows, widgets, etc. Graphical User Interfaces (GUIs) may be employed, including, without limitation, Apple® Macintosh® operating systems' Aqua®, IBM® OS/2®, Microsoft® Windows® (e.g., Aero, Metro, etc.), web interface libraries (e.g., ActiveX®, Java®, Javascript®, AJAX, HTML, Adobe® Flash®, etc.), or the like.

In some embodiments, the computer system **400** may implement the web browser **408** stored program components. The web browser **408** may be a hypertext viewing application, such as MICROSOFT® INTERNET EXPLORER®, GOOGLE™ CHROME™, MOZILLA® FIREFOX®, APPLE® SAFARI®, etc. Secure web browsing may be provided using Secure Hypertext Transport Protocol (HTTPS), Secure Sockets Layer (SSL), Transport Layer Security (TLS), etc. Web browsers **408** may utilize facilities such as AJAX, DHTML, ADOBE® FLASH®, JAVASCRIPT®, JAVA®, Application Programming Interfaces (APIs), etc. In some embodiments, the computer system **400** may implement a mail server stored program component. The mail server may be an Internet mail server such as Microsoft Exchange, or the like. The mail server may utilize facilities such as Active Server Pages (ASP), ACTIVEX®, ANSI® C++/C#, MICROSOFT®, .NET, CGI SCRIPTS, JAVA®, JAVASCRIPT®, PERL®, PHP, PYTHON®, WEBOBJECTS®, etc. The mail server may utilize communication protocols such as Internet Message Access Protocol (IMAP), Messaging Application Programming Interface (MAPI), MICROSOFT® exchange, Post Office Protocol (POP), Simple Mail Transfer Protocol (SMTP), or the like. In some embodiments, the computer system **400** may implement a mail client stored program component. The mail client may be a mail viewing application, such as APPLE® MAIL, MICROSOFT® ENTOURAGE®, MICROSOFT® OUTLOOK®, MOZILLA® THUNDERBIRD®, etc.

Furthermore, one or more computer-readable storage media may be utilized in implementing embodiments consistent with the present invention. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., non-transitory. Examples include Random Access Memory (RAM), Read-Only Memory (ROM), volatile memory, non-volatile memory, hard drives, Compact Disc (CD) ROMs, Digital Video Disc (DVDs), flash drives, disks, and any other known physical storage media.

**Advantages of the present disclosure:**

The present disclosure enables estimation of distance between distal end of an optical fiber and a target, by using laser light of two different wavelengths having a low and high water absorption coefficients. Estimation of the distance based on such wavelength selection, ensures robustness with respect to different types of targets, target compositions, target colors, target surfaces and the like.

The wavelength modulation based technique disclosed in the present disclosure to estimate distance between distal end of an optical fiber and a target, helps in an accurate estimation of the distance, as the process of estimation is robust with respect to various types of targets as mentioned above.

Thus, the present disclosure enables aiming at the target accurately, which in turn eliminates ablating/fragmenting incorrect portion, which could lead to permanent damages, and also consumes less time in ablating/fragmenting the target.

**Applications of the present disclosure:**

The present disclosure may be used to see through the dust. In this Application, during treatment of, for example kidney stones, water may get turbid due to the presence of stone fragments or dust. This may reduce (or cancel at all) the ability to see the target (e.g. stone). Therefore, in such scenarios, the Fiber Feedback (FFB) technique discussed in the present disclosure enables accurately recognizing and informing the treating physician about placement of the optical fiber i.e. if the fiber is placed in front of the target or that there is no target or the fiber is aiming at unwanted area instead of the target.

Further, the present disclosure may be used for distance measurement and target recognition. In this Application, the target (eg: stone) may move around during treatment, which may lead to applying laser towards unwanted area such as healthy tissue, instead of applying laser to the target. Therefore, the present disclosure enables automatic and real-time monitoring of the distance between the optical fiber and the target, which in turn eliminates the possibility of lasing unwanted areas.

Further, the present disclosure may be used for the purpose of smart lasing. In this Application, during the treatment, the target may move back and forth, or may change its shape and size. Therefore, parameters pre-set for the laser sources before initiating lazing on the target, may become less effective. Conventionally, such pre-set parameters are manually changed which may be error prone and time consuming, or in some cases the pre-set parameters may be left unchanged which may lead to scenarios where the optical fiber may be too close or too far from the target. Therefore, the automatic and real-time monitoring of the distance between the optical fiber and the target, as disclosed in the present disclosure, enables automatically changing the lasing pre-set parameters to adjust the lasing in accordance with the target shape, position etc., for best results (i.e. smart lasing).

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended. For example, as an aid to understanding, the detail description may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation *is* explicitly recited, those skilled in the art will recognize that such recitation should typically be interpreted to mean *at least* the recited number (e.g., the bare recitation of "two recitations," without other modifiers, typically means *at least* two recitations, or *two or more* recitations).

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following detailed description.

The following aspects are preferred embodiments of the present invention.

1. A system for accurately estimating a distance between a fiber end and a target, the system comprising:

    an optical fiber, the optical fiber configured to deliver laser light beams originating from a plurality of laser light sources to a target, and is configured to deliver laser light beams reflected from the target to one or more light detectors;

    a Light Emitting, Transmitting and Detecting (LETD) system comprising:

        (i) the plurality of laser light sources comprising a first polarized laser source (L1) and a second polarized laser source (L2) having a wavelength with water absorption coefficient different from that of the first polarized laser source (L1), and one or more other laser sources, the plurality of laser light sources configured to generate incident laser light beams for treating the target;

        (ii) a first beam splitter configured to receive incident laser light beams from the first polarized laser source (L1) and the second polarized laser source (L2), and configured to align the incident laser light beams along a single optical path;

        (iii) a polarizer configured to receive the aligned incident laser light beams from the first beam splitter, and to output polarized laser light beams;

(iv) a first beam combiner configured to:

(a) receive the polarized laser light beams from the polarizer;

(b) combine the polarized laser light beams with an aiming beam and a treatment beam received from the one or more other laser sources; and

(c) output combined laser light beams;

(v) a second beam splitter configured to:

(a) receive the combined laser light beams from the first beam combiner;

(b) align the combined laser light beams along a single optical path;

(c) deliver the aligned combined laser light beams of the second beam splitter to the target via the optical fiber;

(d) receive reflected light via the optical fiber, upon delivering the aligned combined laser light beams of the second beam splitter to the target;

(e) align laser light beams of the reflected light along a single optical path; and

(f) transmit the aligned laser light beams of the reflected light to a polarized beam splitter;

(vi) a polarized beam splitter configured to receive the aligned laser light beams of the reflected light from the second beam splitter and to split the aligned laser light beams of the reflected light into reflected S-Polarized and transmitted P-polarized beams;

(vii) a first light detector configured to:

(a) detect and measure intensity of the transmitted P-polarized beams of the reflected light;

(b) transmit the measured intensity of the transmitted P-polarized beams of the reflected light to a processing unit associated with the LETD system; and (viii) a second light detector configured to:

(a) detect and measure intensity of the reflected S-polarized beams of the reflected light; and

(b) transmit the measured intensity of the reflected S-polarized beams of the reflected light to the processing unit; and

the processing unit configured to:

(i) receive the measured intensities of the transmitted P-polarized beams and reflected S-polarized beams of the reflected light from the first light detector and the second light detector, respectively; and

(ii) estimate a distance between a distal end of the optical fiber and the target based on the measured intensities, the water absorption coefficients of the respective wavelengths of the plurality of laser light sources, and a target reflection coefficient.

2. The system of aspect 1 further comprises a power detector associated with the first beam splitter configured to measure optical power of the incident laser light beams generated by the first polarized laser source (L1) and the second polarized laser source (L2).

3. The system of aspect 1 further comprises an indicator associated with the processing unit configured to indicate the estimated distance between the distal end of the optical fiber and the target, wherein the indicator comprises at least one of visual indicator, audio indicator and a haptic indicator.

4. The system of aspect 1, wherein the wavelength of the first laser light source has a higher water absorption coefficient than the wavelength of the second laser light source.

5. The system of aspect 1, wherein the wavelength of the first laser light source and second laser light source are predefined, and are selected such that the wavelengths are proximal on a wavelength scale.

6. The system of aspect 1, wherein the laser light beams of the reflected light comprises at least one of light beams reflected from the target, light beams reflected from region around the target, and light beams reflected from the distal end of the optical fiber.

7. A system for accurately estimating a distance between a fiber end and a target, the system comprising:

an optical fiber, the optical fiber configured to:

(i) deliver laser light beams originating from a plurality of laser light sources to a target; and
(ii) deliver laser light beams reflected from the target to one or more light detectors;

a Light Emitting, Transmitting and Detecting (LETD) system comprising:

(i) the plurality of laser light sources comprising a first polarized laser source (L1) and a second polarized laser source (L2) having a wavelength with water absorption coefficient different from that of the first polarized laser source (L1), and one or more other laser sources, the plurality of laser light sources configured to generate laser light beams for treating the target;
(ii) a second beam combiner configured to receive incident laser light beams from the first polarized laser source (L1) and the second polarized laser source (L2), and to combine the incident laser light beams;
(iii) a polarizer configured to:

(a) receive the combined incident laser light beams from the second beam combiner; and
(b) output polarized laser light beams;

(iv) a first beam combiner configured to:

(a) receive the polarized laser light beams from the polarizer ;(b) combine the polarized laser light beams with an aiming beam and a treatment beam received from the one or more other laser sources; and
(c) output combined laser light beams;

(v) a second beam splitter configured to:

(a) receive the combined laser light beams from the first beam combiner ;
(b) align the combined laser light beams along a single optical path;
(c) deliver the aligned combined laser light beams of the second beam splitter to the target via the optical fiber;
(d) receive reflected light, via the optical fiber, upon delivering the aligned combined laser light beams of the second beam splitter to the target; and
(e) align laser light beams of the reflected light along a single optical path, and transmit the aligned laser light beams of the reflected light to a polarized beam splitter;

(vi) the polarized beam splitter configured to:

(a) receive the aligned laser light beams of the reflected light from the second beam splitter; and
(b) split the aligned laser light beams of the reflected light into reflected S-Polarized and transmitted P-polarized beams;

(vii) a first light detector configured to:

(a) detect and measure intensity of the transmitted P-polarized beams of the reflected light;
(b) transmit the measured intensity of the transmitted P-polarized beams of the reflected light to a processing unit associated with the LETD system; and

(viii) a second light detector configured to:

(a) detect and measure intensity of the reflected S-polarized beams of the reflected light; and
(b) transmit the measured intensity of the reflected S-polarized beams of the reflected light to the processing unit; and

the processing unit configured to:

(i) receive the measured intensities of the transmitted P-polarized beams and reflected S-polarized beams of the reflected light from the first light detector and the second light detector, respectively; and
(ii) estimate a distance between a distal end of the optical fiber and the target based on the measured intensities, the water absorption coefficients of the respective wavelengths of the plurality of laser light sources, and a target reflection coefficient.

8. The system of aspect 7 further comprises a power detector associated with the second beam splitter configured to measure optical power of the combined laser light comprising the incident laser light beams generated by the first polarized laser source (L1), the second polarized laser source (L2), and the one or more other lasers.

9. The system of aspect 7 further comprises an indicator associated with the processing unit, wherein the indicator is configured to indicate the estimated distance between the distal end of the optical fiber and the target, wherein the indicator comprises at least one of visual indicator, audio indicator and a haptic indicator.

10. The system of aspect 6, wherein the wavelength of the first laser light source has a higher water absorption coefficient than the wavelength of the second laser light source.

11. The system of aspect 1, wherein the wavelength of the first laser light source and second laser light source are predefined, and are selected such that the wavelengths are proximal on a wavelength scale.

12. The system of aspect 6, wherein the laser light beams of the reflected light comprises at least one of light beams reflected from the target, light beams reflected from region around the target, and light beams reflected from the distal end of the optical fiber.

13. A system for accurately estimating a distance between a fiber end and a target, the system comprising:

an optical fiber, the optical fiber configured to:

(i) deliver laser light beams originating from a plurality of laser light sources, to a target; and
(ii) deliver laser light beams reflected from the target, to one or more light detectors;

a Light Emitting, Transmitting and Detecting (LETD) system comprising:

(i) the plurality of laser light sources comprising a first non-polarized laser source (L1') and a second non-polarized laser source (L2') having a wavelength with water absorption coefficient different from that of the first non-polarized laser source (L1'), and one or more other laser sources, the plurality of laser light sources configured to generate incident laser light beams for treating the target;
(ii) a first beam splitter configured to:

(a) receive incident laser light beams from the first non-polarized laser source (L1') and the second non-polarized laser source (L2'); and
(b) align the incident laser light beams along a single optical path;

(iii) a first beam combiner configured to:

(a) receive the aligned incident laser light beams from the first beam splitter;
(b) combine the aligned incident laser light beams with an aiming beam and a treatment beam received from the one or more other laser sources; and
(c) output combined laser light beams;

(iv) a second beam splitter configured to:

(a) receive the combined laser light beams from the first beam combiner;
(b) align the combined laser light beams along a single optical path;
(c) deliver the aligned combined laser light beams of the second beam splitter to the target via the optical fiber;
(d) receive reflected light, via the optical fiber, upon delivering the aligned combined laser light beams of the second beam splitter to the target; and
(e) align laser light beams of the reflected light along a single optical path, and transmit the aligned laser light beams of the reflected light to a third light detector;

(v) the third light detector configured to:

(a) detect and measure intensity of the aligned laser light beams of the reflected light; and
(b) transmit the measured intensity to a processing unit associated with the LETD system; and

the processing unit configured to:

(i) receive the measured intensity of the aligned laser light beams of the reflected light from the third light detector; and
(ii) estimate a distance between a distal end of the optical fiber and the target based on the measured intensity, the water absorption coefficients of the respective wavelengths of the plurality of laser light sources, and a target reflection coefficient.

14. The system of aspect 13 further comprises a power detector associated with the first beam splitter configured to measure optical power of the laser light beams generated by the first non-polarized laser source (L1') and the second non-polarized laser source (L2').

15. The system of aspect 13 further comprises an indicator associated with the processing unit, wherein the indicator is configured to indicate the estimated distance between the distal end of the optical fiber and the target, wherein the indicator comprises at least one of visual indicator, audio indicator and a haptic indicator.

16. The system of aspect 13, wherein the wavelength of the first laser light source has a higher water absorption coefficient than the wavelength of the second laser light source.

17. The system of aspect 13, wherein the wavelength of the first laser light source and second laser light source are predefined, and are selected such that the wavelengths are proximal on a wavelength scale.

18. The system of aspect 13, wherein the laser light beams of the reflected light comprises at least one of light beams reflected from the target, light beams reflected from region around the target, and light beams reflected from a proximal end and the distal end of the optical fiber.

19. A system for accurately estimating a distance between a fiber end and a target, the system comprising:

an optical fiber, the optical fiber configured to:

(i) deliver laser light beams originating from a plurality of laser light sources to a target; and
(ii) deliver laser light beams reflected from the target, to one or more light detectors;

a Light Emitting, Transmitting and Detecting (LETD) system comprising:

(i) the plurality of laser light sources comprising:

(a) a first polarized laser source (L1);
(b) a second polarized laser source (L2) having a wavelength with water absorption coefficient different from that of the first polarized laser source (L1);
(c) a third polarized laser source (L3) having a wavelength with water absorption coefficient different from that of the first polarized laser source (L1) and the second polarized laser source (L2); and
(d) one or more other laser sources, wherein the plurality of laser light sources are configured to generate incident laser light beams for treating the target;

(ii) a first beam splitter configured to:

(a) receive incident laser light beams from the first polarized laser source (L1), the second polarized laser source (L2) and the third polarized laser source (L3); and
(b)align the incident laser light beams along a single optical path;

(iii) a third beam splitter configured to:

(a) receive the aligned incident laser beams from the first beam splitter; and
(b) align the aligned incident laser light beams of the first beam splitter and the incident laser light beams received from the third polarized laser source (L3) along a single optical path;

(iv) a polarizer configured to:

(a) receive the aligned incident laser light beams from the third beam splitter; and
(b) output the polarized laser light;

(v) a first beam combiner configured to:

(a) receive the polarized laser light beams from the polarizer;
(b) combine the polarized laser light beams with an aiming beam and a treatment beam received from the one or more other laser sources; and
(c) output combined laser light beams;

(vi) a second beam splitter configured to:

(a) align the combined laser light beams from the first beam combiner;
(b)align the combined laser light beams along a single optical path;
(c) deliver the aligned combined laser light beams of the second beam splitter to the target via the optical fiber;
(d) receive reflected light, via the optical fiber, upon delivering the aligned combined laser light beams of the second beam splitter to the target;
(e) align laser light beams of the reflected light along a single optical path; and
(f) transmit the aligned laser light beams of the reflected light to a polarized beam splitter;

(vii) the polarized beam splitter configured to split the aligned laser light beams of the reflected light into reflected S-Polarized and transmitted P-polarized beams;
(viii) a first light detector configured to:

(a) detect and measure intensity of the transmitted P-polarized beams of the reflected light; and
(b) transmit the measured intensity of the transmitted P-polarized beams of the reflected light to a processing unit associated with the LETD system;

(ix) a second light detector configured to:

(a) detect and measure intensity of the reflected S-polarized beams of the reflected light; and
(b) transmit the measured intensity of the reflected S-polarized beams of the reflected light to the processing unit; and

the processing unit configured to:

(i) receive the measured intensities of the transmitted P-polarized beams and reflected S-polarized beams of the reflected light from the first light detector and the second light detector, respectively; and
(ii) estimate a distance between a distal end of the optical fiber and the target based on the measured intensities, the water absorption coefficients of the respective wavelengths of the plurality of laser light sources, and a target reflection coefficient.

20. The system of aspect 19 further comprises a power detector associated with the third beam splitter configured to measure optical power of the laser light beams generated by the first polarized laser source (L1), the second polarized laser source (L2) and the third polarized laser source (L3).

21. The system of aspect 19 further comprises an indicator associated with the processing unit, wherein the indicator is configured to indicate the estimated distance between the distal end of the optical fiber and the target, wherein the indicator comprises at least one of visual indicator, audio indicator and a haptic indicator.

22. The system of aspect 19, wherein the wavelength of the first laser light source (L1) has a higher water absorption than the wavelength of the second laser light source (L2), and the wavelength of the third laser light source (L3) has a higher water absorption than the wavelength of the first laser light source (L1) and the second laser light source (L2).

23. The system of aspect 19, wherein the wavelength of the first laser light source and second laser light source are predefined and are selected such that the wavelengths are proximal on a wavelength scale.

24. The system of aspect 19, wherein the laser light beams of the reflected light comprises at least one of light beams reflected from the target, light beams reflected from region around the target, and light beams reflected from a proximal end and the distal end of the optical fiber.

25. A system for accurately estimating a distance between a fiber end and a target, the system comprising:

an optical fiber, the optical fiber configured to deliver laser light beams originating from a plurality of laser light sources to a target, and laser light beams reflected from the target to one or more light detectors;
a Light Emitting, Transmitting and Detecting (LETD) system comprising:

(i) the plurality of laser light sources comprising a first polarized laser source (L1), a second polarized laser source (L2) having a wavelength with water absorption coefficient different from that of the first polarized laser source (L1), a third polarized laser source (L3) having a wavelength with water absorption coefficient different from that of the first polarized laser source (L1) and the second polarized laser source (L2), and one or more other laser sources, the plurality of laser light sources configured to generate incident laser light beams for treating the target;
(ii) a Wavelength Division Multiplexer (WDM) configured to:

(a) receive incident laser light beams from the first polarized laser source (L1), the second polarized laser source (L2), the third polarized laser source (L3), and an aiming beam generated by the one or more other laser sources; and
(b) align the incident laser light beams along a single optical path;

(iii) a fourth beam splitter configured to:

(a) receive the aligned incident laser light beams from the WDM; and
(b) align incident laser light beams of the WDM along a single optical path;

(iv) a collimator configured to:

(a) receive the aligned incident laser light beams from the fourth beam splitter; and
(b) narrow down the aligned output laser light beams into parallel laser light beams;

(v) a first beam combiner configured to:

(a) receive the parallel laser light beams from the polarizer;
(b) combine the parallel light beams with another aiming beam and a treatment beam received from the one or more other laser sources; and
(c) output combined laser light beams;

(vi) a second beam splitter configured to:

(a) receive the combined laser light beams;
(b)align the combined laser light beams along a single optical path;
(c) deliver the aligned combined laser light beams of the second beam splitter to the target via the optical fiber;
(d) receive reflected light, via the optical fiber, upon delivering the aligned combined laser light beams of the second beam splitter to the target;
(e) align laser light beams of the reflected light along a single optical path; and
(f) transmit the aligned light beams of the reflected light to a third light detector;

(vii) the third light detector configured to:

(a) detect and measure intensity of the aligned laser light beams of the reflected light; and
(b) transmit the measured intensity of the aligned laser light beams of the reflected light to a processing unit associated with the LETD system; and

the processing unit configured to:

(i) receive the measured intensity of the aligned laser light beams of the reflected light; and
(ii) estimate a distance between a distal end of the optical fiber and the target based on the measured intensity, the water absorption coefficients of the respective wavelengths of the plurality of laser light sources, and a target reflection coefficient.

26. The system of aspect 25 further comprises a power detector associated with the fourth beam splitter configured to measure optical power of the laser light beams generated by the first polarized laser source (L1), the second polarized laser source (L2), the third polarized laser source (L3) and the one or more other laser sources.

27. The system of aspect 25 further comprises an indicator associated with the processing unit, wherein the indicator is configured to indicate the estimated distance between the distal end of the optical fiber and the target, wherein the indicator comprises at least one of visual indicator, audio indicator and a haptic indicator.

28. The system of aspect 25, wherein the wavelength of the first laser light source (L1) has a higher water absorption coefficient than the wavelength of the second laser light source (L2), and the wavelength of the third laser light source (L3) has a higher water absorption co-efficient than the wavelength of the first laser light source (L1) and the second laser light source (L2).

29. The system of aspect 25, wherein the wavelength of the first laser light source (L1), the second laser light source (L2) and the third laser light source (L3) are predefined and are selected such that the wavelengths are proximal on a wavelength scale.

30. The system of aspect 25, wherein the laser light beams of the reflected light comprises at least one of light beams reflected from the target, light beams reflected from region around the target, and light beams reflected from a proximal end and the distal end of the optical fiber.

31. A system for accurately estimating a distance between a fiber end and a target, the system comprising:

an optical fiber, the optical fiber configured to deliver laser light beams originating from a plurality of laser light sources to a target, and laser light beams reflected from the target to one or more light detectors;
a Light Emitting, Transmitting and Detecting (LETD) system comprising:

(i) the plurality of laser light sources comprising a first polarized laser source (L1), a second polarized laser source (L2) having a wavelength with water absorption coefficient different from that of the first polarized laser source (L1), a third polarized laser source (L3) having a wavelength with water absorption coefficient different from that of the first polarized laser source (L1) and the second polarized laser source (L2), and one or more other laser sources, the plurality of laser light sources configured to generate incident laser light beams for treating the target;
(ii) a Wavelength Division Multiplexer (WDM) configured toreceive incident laser light beams from the first polarized laser source (L1), the second polarized laser source (L2), the third polarized laser source (L3), and an aiming beam generated by the one or more other laser sources, and to align the incident laser light beams along a single optical path;
(iii) a fourth beam splitter configured to:

(a) receive the aligned incident laser light beams from the WDM; and

(b) align incident laser light beams of the WDM along a single optical path;

(iv) a circulator configured to:

(a) receive the aligned incident laser light beams from the fourth beam splitter; and
(b) enable the aligned incident laser light beams to travel in a single direction;

(v) a collimator configured to:

(a) receive the aligned incident laser light beams from the circulator; and
(b) narrow down the aligned laser light beams received from the circulator into parallel laser light beams;

(vi) a first beam combiner configured to:

(a) receive the parallel laser light beams from the polarizer;
(b) to combine the parallel light beams with another aiming beam and a treatment beam received from the one or more other laser sources;
(c) output combined laser light beams; and
(d) deliver the combined laser light beams of the first beam combiner to the target via the optical fiber;

(vii) the circulator configured to:

(a) receive reflected light, via the optical fiber, upon delivering the combined laser light beams of the first beam combiner to the target;
(b) enable the laser light beams of the reflected light to travel in a single direction;
(c) transmit laser light beams of the reflected light coming from the collimator, to a third light detector;

(viii) the third light detector configured to:

(a) detect and measure intensity of the aligned laser light beams of the reflected light received from the collimator; and
(b) transmit the measured intensity of the aligned laser light beams of the reflected light to a processing unit associated with the LETD system; and

the processing unit configured to:

(i) receive the measured intensity of the aligned laser light beams of the reflected light; and
(ii) estimate a distance between a distal end of the optical fiber and the target based on the measured intensity, the water absorption coefficients of the respective wavelengths of the plurality of laser light sources, and a target reflection coefficient.

32. The system of aspect 31 further comprises a power detector associated with the fourth beam splitter configured to measure optical power of the laser light beams generated by the first polarized laser source (L1), the second polarized laser source (L2), the third polarized laser source (L3) and the one or more other laser sources.

33. The system of aspect 31 further comprises an indicator associated with the processing unit, wherein the indicator is configured to indicate the estimated distance between the distal end of the optical fiber and the target, wherein the indicator comprises at least one of visual indicator, audio indicator and a haptic indicator.

34. The system of aspect 31, wherein the wavelength of the first laser light source (L1) has a higher water absorption coefficient than the wavelength of the second laser light source (L2), and the wavelength of the third laser light source (L3) has a higher water absorption co-efficient than the wavelength of the first laser light source (L1) and the second laser light source (L2).

35. The system of aspect 31, wherein the wavelength of the first laser light source (L1), the second laser light source (L2) and the third laser light source (L3) are predefined and are selected such that the wavelengths are proximal on a wavelength scale.

36. The system of aspect 31, wherein the laser light beams of the reflected light comprises at least one of light beams reflected from the target, light beams reflected from region around the target, and light beams reflected from a proximal end and the distal end of the optical fiber.

37. The system of any one of aspects 19, 25 or 31, wherein the third polarized laser source (L3) is configured for calibration of an optical fiber condition.

38. The system of aspect 37, wherein the optical fiber condition is the optical quality of the distal end of the optical fiber.

39. The system of aspect 37, wherein the calibration is done in real time, and a deviation from the calibration indicates a degradation in the optical condition of the distal end of the optical fiber.

40. The system of aspect 39, further comprising a second indicator associated with the processing unit configured to indicate to a user a degradation of the optical condition of the distal end of the optical fiber.

41. The system of any of the preceding aspects, wherein the proximal end of the optical fiber is coated with an anti-reflective coat to reduce noise caused at the proximal end of the optical fiber and to increase dynamic range of the reflected light received from the target.

42. The system of any of the preceding aspects, wherein the proximal end of the optical fiber is cut at a predefined angle to enable diversion of unwanted reflected light from the proximal end of the optical fiber.

43. The system of any one of aspects 1, 8, 14, 19, 25 or 31, wherein the first beam combiner is configured to receive from the one or more other laser sources at least one non-polarized treatment beam and to output the combined laser light beam.

44. A method of estimating distance between a fiber end and a target, the method comprising:

providing a Light Emitting, Transmitting and Detecting (LETD) system comprising a plurality of laser light sources and a plurality of light detectors;
providing a processing unit;
illuminating, by the LETD system, a target with incident laser light beams of plurality of wavelengths, via an optical fiber, using at least one of the plurality of laser light sources;
receiving, by the LETD system, via the optical fiber and the plurality of light detectors, reflected light from the target;
measuring, by the LETD system, via the light detectors, intensities of light beams of the reflected light of each of the plurality of wavelengths;
transmitting, by the LETD system, the measured intensities to a processing unit,
wherein the processing unit receives the measured intensities of the light beams of the reflected light of at least two of the plurality of wavelengths, from the LETD system, and estimates a distance between a distal end of the optical fiber and the target based on the measured intensities, a water absorption coefficients of the respective plurality of wavelengths, and a target reflection coefficient.

45. A method of estimating distance between a fiber end and a target, the method comprising:

providing a Light Emitting, Transmitting and Detecting (LETD) system comprising a plurality of laser light sources having a plurality of wavelengths and a plurality of light detectors;
providing a processing unit;
receiving, by the processing unit, measured intensities of light beams of reflected light of at least two of the plurality of wavelengths, from the LETD system, wherein the LETD system is configured to:

(i) illuminate a target with incident laser light beams of the plurality of wavelengths, via an optical fiber, using at least one of the plurality of laser light sources; and
(ii) measure intensities of the light beams of the reflected light of each of the plurality of wavelengths; and

estimating, by the processing unit, a distance between a distal end of the optical fiber and the target based on the measured intensities, a water absorption coefficients of the respective plurality of wavelengths, and a target reflection coefficient.

46. The method as claimed in aspect 45, wherein estimating the distance between the distal end of the optical fiber and the target comprises:
determining, by the processing unit, a ratio of the measured intensities of the light beams of the reflected light belonging to two different wavelengths of the plurality of wavelengths, wherein the two different wavelengths belong to one of a first polarized laser source (L1) and a second polarized laser source (L2), or a first non-polarized laser source (L1') and a second non-polarized laser source (L2'), using an equation:

$$\frac{I_{(HI)}}{I_{(LO)}} = \frac{R}{R} * \ e^{(\lambda_{LO} - \lambda_{HI}) * X}$$

wherein,

$I_{(HI)}$ and $I_{(LO)}$ are the measured intensities of light beams corresponding to the two different wavelengths respectively, $\frac{I_{(HI)}}{I_{(LO)}}$ the ratio of the measured intensities of the light beams of the reflected light belonging to the two different wavelengths,
**R** is the target reflection coefficient,
$\lambda_{LO}$ and $\lambda_{HI}$ are water absorption coefficients of the two different wavelengths respectively,
**X** is the distance between the distal end of the optical fiber and the target; and

estimating, by the processing unit, the distance **(X)** between the distal end of the optical fiber and the target based on the ratio of the measured intensities ($I_{(HI)}$ and $I_{(LO)}$) of the two different wavelengths, the water absorption coefficients of the two different wavelengths ($\lambda_{LO}$ and $\lambda_{HI}$), and the target reflection coefficient **(R)** using the equation:

$$X = \frac{\ln\left(\frac{I_{(HI)}}{I_{(LO)}}\right)}{\lambda_{LO} - \lambda_{HI}}$$

wherein "In" is a natural logarithm.

47. The method of aspects 44 or 45 further comprises indicating, by an indicator associated with the processing unit, the estimated distance between the distal end of the optical fiber and the target, wherein the indicator comprises at least one of a visual indicator, an audio indicator and a haptic indicator.

48. The method of aspects 44 or 45 further comprises measuring, by a power detector, an optical power of the laser light beams generated by the plurality of laser light sources.

49. The method of aspects 44-48 are performed using at least one of the systems disclosed in the preceding claims 1, 7, 13, 19, 25 and 31.

50. The method of aspects 45 or 46, further comprising:

receiving, by the processing unit from the LETD system, measured intensities of light beams belonging to each of at least three of the plurality of wave lengths of the reflected light under a "no target condition",
wherein the LETD system is configured to:

(i) illuminate a target under the "no target condition" with incident laser light beams of a plurality of wavelengths, via an optical fiber, using at least three of a plurality of laser light sources;
(ii) receive the reflected light of the incident laser light beams of each of at least three of the plurality of wavelengths, via the optical fiber;
(iii) measure intensities of the reflected light of the incident laser light beams of each of the at least three of a plurality of wavelengths; and
(iv) transmit the measured intensities of the reflected light of the incident laser light beams of each of the at least three of a plurality of wavelengths to the processing unit.

storing, by the processing unit, the measured intensities of the reflected light of incident laser light beams of each of the at least three of a plurality of wavelengths as an internal reflection pre-treatment value (IR cal-pre).

51. The method of aspect 50, wherein the plurality of laser light sources comprises: a first polarized laser source (L1) having a wavelength with a high water absorption coefficient (HI), a second polarized laser source (L2) with a low water absorption coefficient (LO), a third polarized laser source (L3) having a wavelength with a higher water absorption coefficient than the first polarized laser source (L1).

52. The method of aspect 51, wherein:

the water absorption coefficient of the first polarized laser source (L1) is higher than the water absorption coefficient of the second polarized laser source (L2); and
the water absorption co-efficient of the third polarized laser source (L3) is higher than the water absorption coefficient of the first polarized laser source (L1), and the water absorption coefficient of the second polarized laser source (L2).

53. The method of aspect 50, wherein the plurality of laser light sources comprises: a first non-polarized laser source (L1') having a wavelength with the high water absorption coefficient (HI), a second non-polarized laser source (L2') with the low water absorption coefficient (LO), a third non-polarized laser source (L3') having the wavelength with the higher water absorption coefficient than the first non-polarized laser source (L1'), and one or more other laser sources.

54. The method of aspect 53, wherein:

the water absorption coefficient of the first non-polarized laser source (L1') is higher than the water absorption coefficient of the second non-polarized laser source (L2'); and
the water absorption co-efficient of the third non-polarized laser source (L3') is higher than the water absorption coefficient of the first non-polarized laser source (L1'), and the second non-polarized laser source (L2').

55. The method of aspects 52 or 54, further comprises:

receiving, by the processing unit from the LETD system, new measured intensities of light beams of reflected light belonging to each of the plurality of wavelengths;

determining, by the processing unit, a calibration factor based on at least one of the following:

(i) the stored internal reflection pre-treatment value ($IR_{\mathbf{CAL\text{-}PRE}}$) of a third polarized laser light source (L3), and the new measured intensity **($IR_{\mathbf{CAL\text{-}DUR}}$)** of the light beams of the reflected light of the wavelength belonging to the third polarized laser light source (L3); or

(ii) the stored internal reflection pre-treatment value ($IR_{\mathbf{CAL\text{-}PRE}}$) of a third non-polarized laser light source (L3'), and the new measured intensity **($IR_{\mathbf{CAL\text{-}DUR}}$)** of the light beams of the reflected light of the wavelength belonging to the third non-polarized laser light source (L3').

56. The method of aspect 55, wherein determining an optical fiber condition comprises estimating, by the processing unit, distance between the distal end of the optical fiber and the target based on the measured intensities, water absorption coefficients of the respective wavelengths of the plurality of laser light sources, the determined calibration factor and the target reflection coefficient, thereby calibrating the optical fiber condition.

57. The method of aspect 56, wherein calibrating and estimating distance between the distal end of the optical fiber and the target in real time comprises:

determining, by the processing unit, new calibrated intensities of the light beams of the reflected light belonging to two different wavelengths of the plurality of wavelengths, based on the measured intensities of the light beams of the reflected light belonging to the two different wavelengths of the plurality of wavelengths wherein the two different wavelengths belong to one of the first polarized laser source (L1) and the second polarized laser source (L2), or the first non-polarized laser source (L1') and the second non-polarized laser source (L2'), using the equation:

$$\mathbf{I''} = \mathbf{I} - \mathbf{IR} \times \mathbf{CF}$$

wherein, **I''** is the new calibrated intensity of the light beams of the reflected light of a particular wavelength, **I** is the measured intensity of the light beams of the reflected light of the particular wavelength, CF is the determined calibration factor and **IR** is the intensity of the light beams of the reflected light of the particular wavelength measured for the "no target condition";
determining, by the processing unit, a ratio of the new calibrated intensities of the light beams of the reflected light belonging to the two different wavelengths of the plurality of wavelengths, using an equation:

$$\frac{I''_{(HI)}}{I''_{(LO)}} = \frac{R}{R} * e^{(\lambda_{LO} - \lambda_{HI}) * X}$$

wherein,

$I''_{(HI)}$ and $I''_{(LO)}$ are the new calibrated intensities of light beams corresponding to the two different wavelengths respectively, $\frac{I''(HI)}{I''(LO)}$ the ratio of the new calibrated intensities of the light beams of the reflected light belonging to the two different wavelengths,
**R** is the target reflection coefficient,
$\lambda_{LO}$ and $\lambda_{HI}$ are water absorption coefficients of the two different wavelengths respectively,
**X** is the distance between the distal end of the optical fiber and the target; and

estimating, by the processing unit, the distance between the distal end of the optical fiber and the target **(X)** based on the ratio of the new calibrated intensities ($I''_{(HI)}$ and $I''_{(LO)}$) of the two different wavelengths, the water absorption coefficients of the two different wavelengths ($\lambda_{LO}$ and $\lambda_{HI}$), and the target reflection coefficient **(R)** using the equation:

$$X = \frac{\ln\left(\frac{I''_{(HI)}}{I''_{(LO)}}\right)}{\lambda_{LO} - \lambda_{HI}}$$

wherein "ln" is a natural logarithm.

58. The method of aspect 55, the calibration factor is determined using the equation:

$$CF = \frac{IR_{(CAL-PRE)}}{IR_{(CAL-DUR)}}$$

wherein, CF is the calibration factor.

59. The method of aspects 50-58 are performed using at least one of the systems disclosed in the preceding claims 19, 25, and 31.

## Claims

**1.** A system (105) for estimating a distance between an end of an optical fiber and a target, the system comprising:

a first laser light source (L1) having a wavelength with a first water absorption coefficient (HI);
a second laser light source (L2) having a wavelength with a second water absorption coefficient (LO) different from that of the first water absorption coefficient (HI);

a beam splitter (211) configured to receive a portion of the laser light beams generated by the first laser light source (L1) and the second laser light source (L2);

a port (219) optically associated with the second beam splitter (211), the port configured to optically couple an optical fiber (103) with the beam splitter (211), wherein the laser light beams generated by the first laser light source (L1) and the second laser light source (L2) can be directed to a target (101) via an end of the optical fiber (103) and reflected light beams can be received via the end of the optical fiber (103) and directed to a light detector (227);

the light detector (227) optically associated with the beam splitter, the light detector (227) configured to measure intensity of the reflected light beams; and

a processor (402) and a memory (405) comprising instructions that when executed by the processor (402) cause the processor (402) to estimate a distance between the end of the optical fiber (103) and the target (101) based on the measured intensity of the reflected light beams and the first water absorption coefficient and the second water absorption coefficient.

**2.** The system of claim 1, wherein the beam splitter (211) is a distal beam splitter, the system further comprising:

a proximal beam splitter (203) configured to receive laser light beams generated by the first laser light source (L1) and the second laser light source (L2); and

a power detector (205) optically associated with the proximal beam splitter (203), the power detector (205) configured to measure optical power of the laser light beams generated by the first laser light source (L1) and the second laser light source (L2).

**3.** The system of any one of claims 1 or 2, further comprises an indicator (109) associated with the processor (402) configured to indicate the estimated distance between the end of the optical fiber (103) and the target (101), wherein the indicator comprises at least one of a visual indicator, an audio indicator, or a haptic indicator.

**4.** The system of any one of claims 1 to 3, wherein the first water absorption coefficient (HI) is higher than the second water absorption coefficient (LO).

**5.** The system of any one of claims 1 to 4, wherein the wavelength of the first laser light source and second laser light source are predefined and are selected such that the wavelengths are proximal on a wavelength scale.

**6.** The system of any one of claims 1 to 5, wherein the reflected light beams comprise light beams reflected from the target (101) or light beams from a region around the target.

**7.** The system of any one of claims 1 to 6, wherein the laser light beams generated by the first laser light source (L1) and the second laser light source (L2) are non-polarized.

**8.** The system of any one of claims 1 to 7, further comprising:

a solid-state laser or a fiber laser configured to generate a treatment beam; and

a beam combiner configured to combine the treatment beam and the light beams generated by the first laser light source (L1) and the second laser light source (L2).

**9.** The system of claim 8, further comprising an aiming beam laser source configured to generate an aiming beam, wherein the beam combiner is further configured to combine the aiming beam with the treatment beam and the light beams generated by the first laser light source (L1) and the second laser light source (L2).

**10.** A method (300) for estimating a distance between an end of an optical fiber and a target by a light emitting, transmitting and detecting (LETD) system 105, the method comprising:

illuminating (301), via an end of an optical fiber (103), a target (101) with a first laser light beam having a wavelength with a first water absorption coefficient (HI) and a second laser light beam having a wavelength with a second water absorption coefficient (LO) different from that of the first water absorption coefficient (HI);

receiving (303), via the end of the optical fiber (103), reflected light beams, wherein the reflected light beams are reflected off the target (101) responsive to illumination by the first laser light beam and the second laser light beam;

measuring (305) intensity of the reflected light beams; and

estimating (309) a distance between the end of the optical fiber (103) and the target (101) based on the measured intensity of the reflected light beams and the first water absorption coefficient and the second water absorption coefficient.

**11.** The method of claim 10, wherein illuminating (301) the target (101) with the first laser light beam and the second laser light beam comprises:

generating, via a first laser light source (L1), the first laser light beam; and
generating, via a second laser light source (L2), the second laser light beam.

**12.** The method of any one of claims 10 to 11, wherein measuring (305) the intensity of the reflected light beams comprises receiving, from a light detector, the measured intensity, wherein the reflected light beams are received at a beam splitter and wherein the beam splitter is configured to direct the reflected light beams to the light detector.

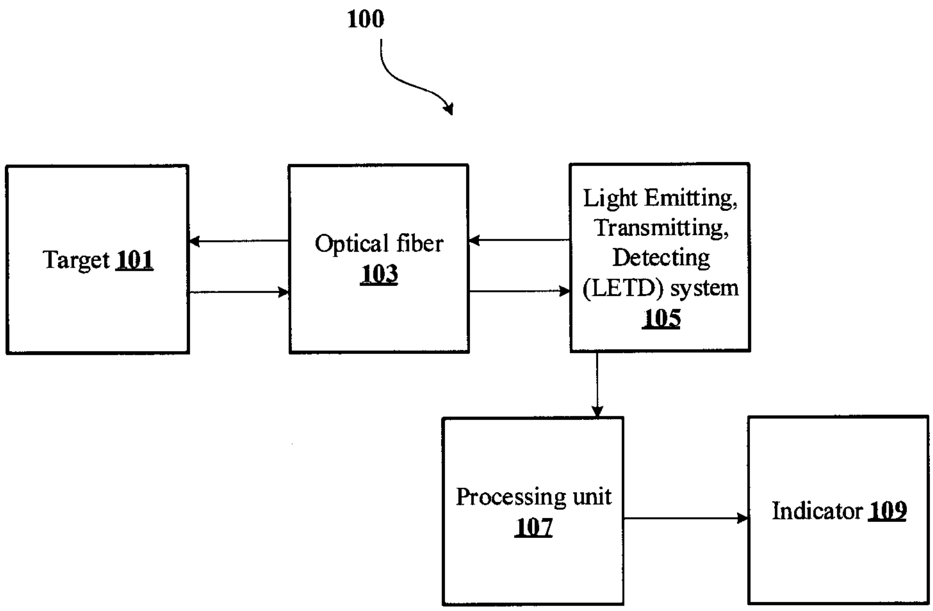
100
Target 101
Optical fiber 103
Light Emitting, Transmitting, Detecting (LETD) system 105
Processing unit 107
Indicator 109

FIG.1A

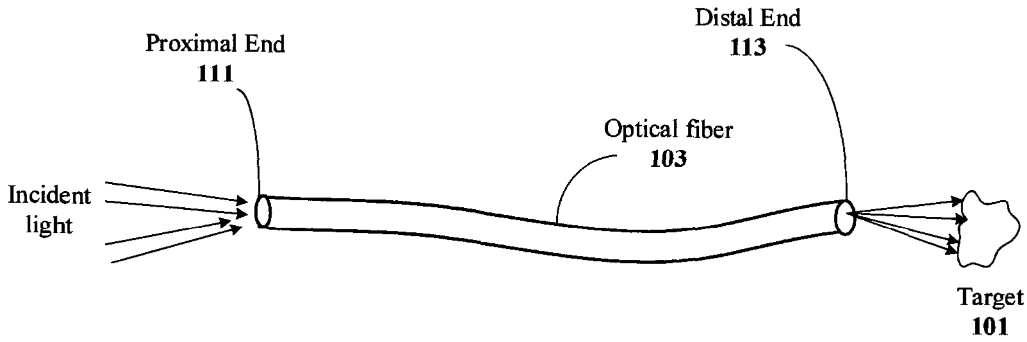
Proximal End
111
Distal End
113
Optical fiber
103
Incident
light
Target
101

FIG.1B

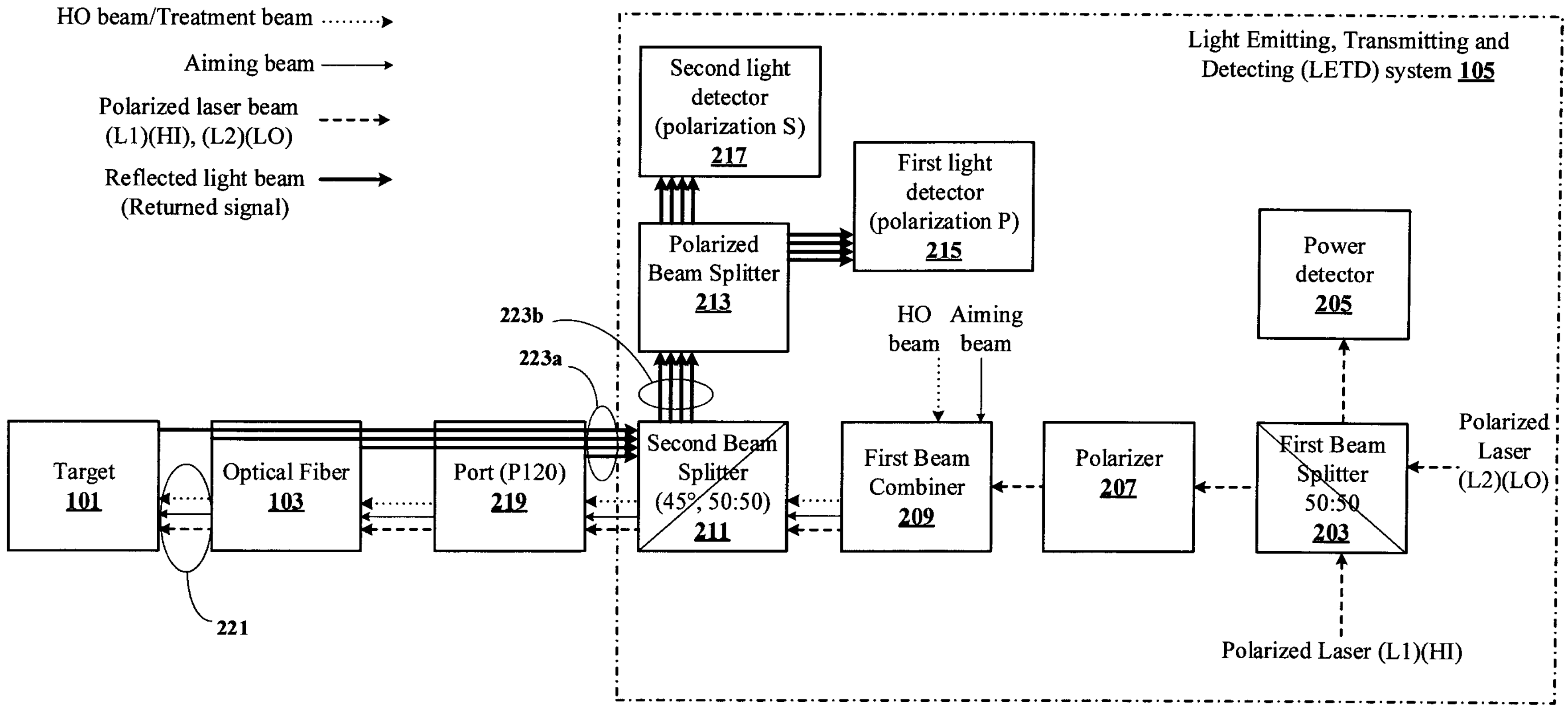
HO beam/Treatment beam
Aiming beam
Polarized laser beam (L1)(HI), (L2)(LO)
Reflected light beam (Returned signal)
Light Emitting, Transmitting and Detecting (LETD) system 105
Second light detector (polarization S) 217
First light detector (polarization P) 215
Power detector 205
Polarized Beam Splitter 213
HO beam
Aiming beam
223b
223a
Target 101
Optical Fiber 103
Port (P120) 219
Second Beam Splitter (45°, 50:50) 211
First Beam Combiner 209
Polarizer 207
First Beam Splitter 50:50 203
Polarized Laser (L2)(LO)
Polarized Laser (L1)(HI)
221

FIG.2A

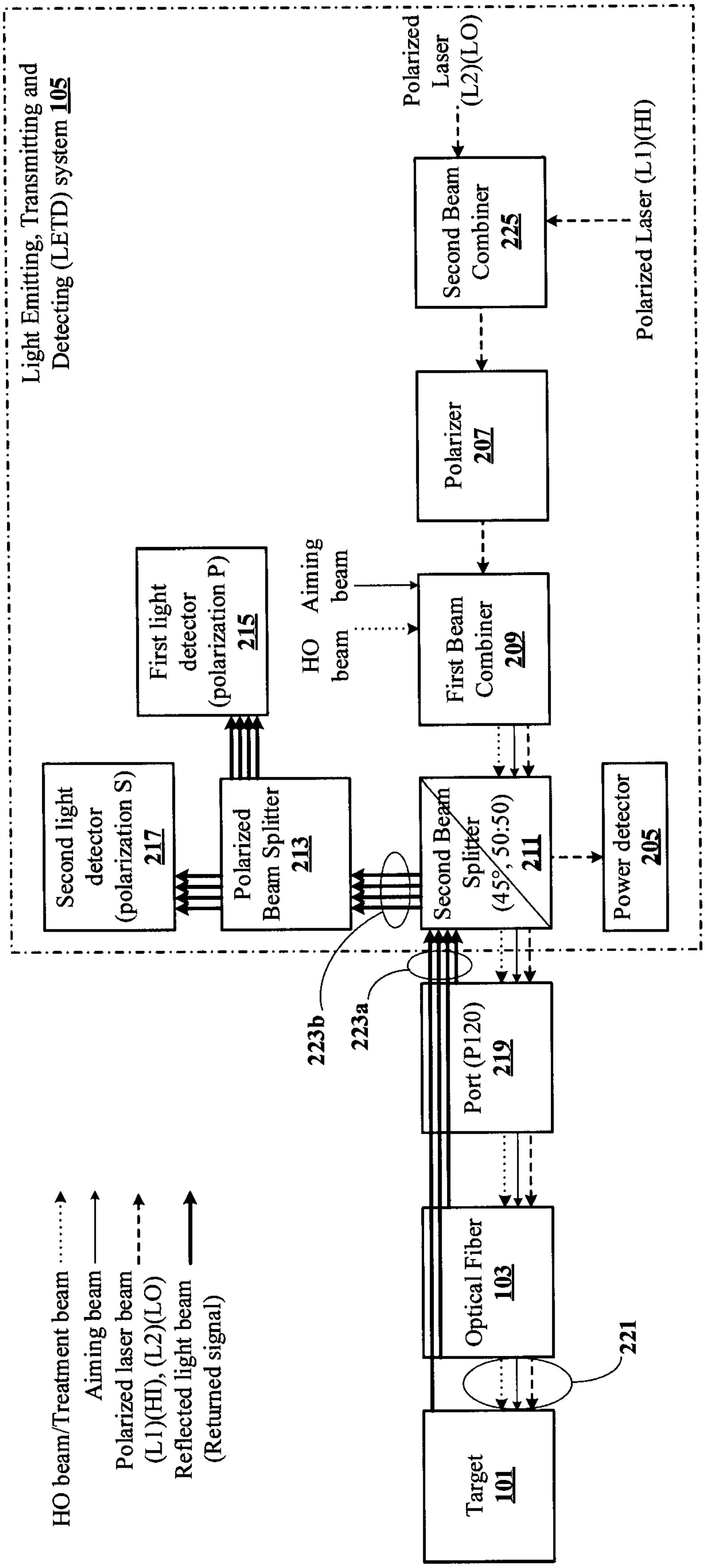
Light Emitting, Transmitting and Detecting (LETD) system 105
Polarized Laser (L2)(LO)
Second Beam Combiner 225
Polarized Laser (L1)(HI)
Polarizer 207
First light detector (polarization P) 215
HO beam
Aiming beam
First Beam Combiner 209
Second light detector (polarization S) 217
Polarized Beam Splitter 213
Second Beam Splitter (45°, 50:50) 211
Power detector 205
223b
223a
Port (P120) 219
Optical Fiber 103
221
Target 101
HO beam/Treatment beam
Aiming beam
Polarized laser beam (L1)(HI), (L2)(LO)
Reflected light beam (Returned signal)

FIG.2B

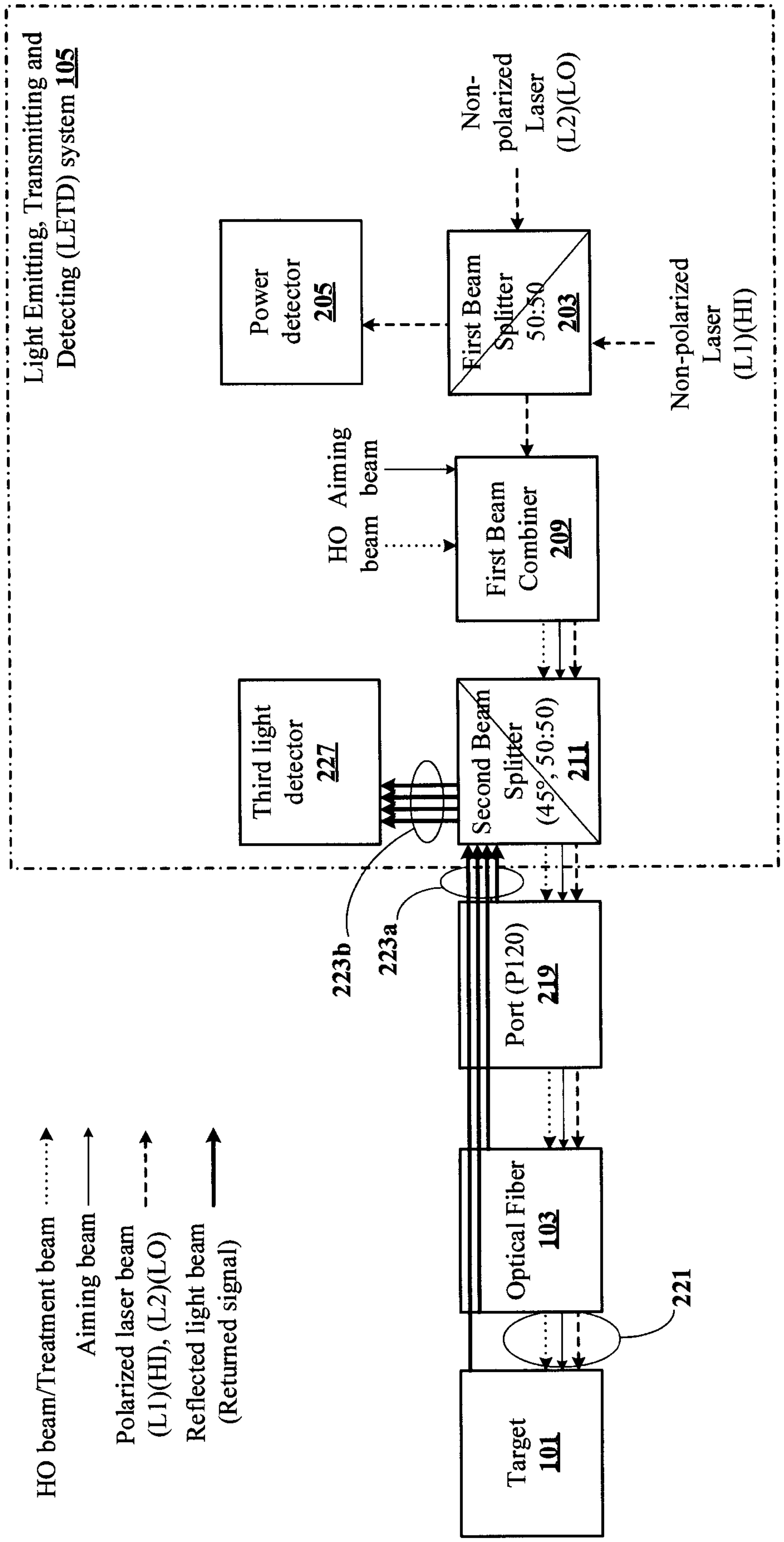
Light Emitting, Transmitting and Detecting (LETD) system 105
Non-polarized Laser (L2)(LO)
Power detector 205
First Beam Splitter 50:50 203
Non-polarized Laser (L1)(HI)
HO beam
Aiming beam
First Beam Combiner 209
Third light detector 227
Second Beam Splitter (45°, 50:50) 211
223b
223a
Port (P120) 219
Optical Fiber 103
221
Target 101
HO beam/Treatment beam
Aiming beam
Polarized laser beam (L1)(HI), (L2)(LO)
Reflected light beam (Returned signal)

FIG.2C

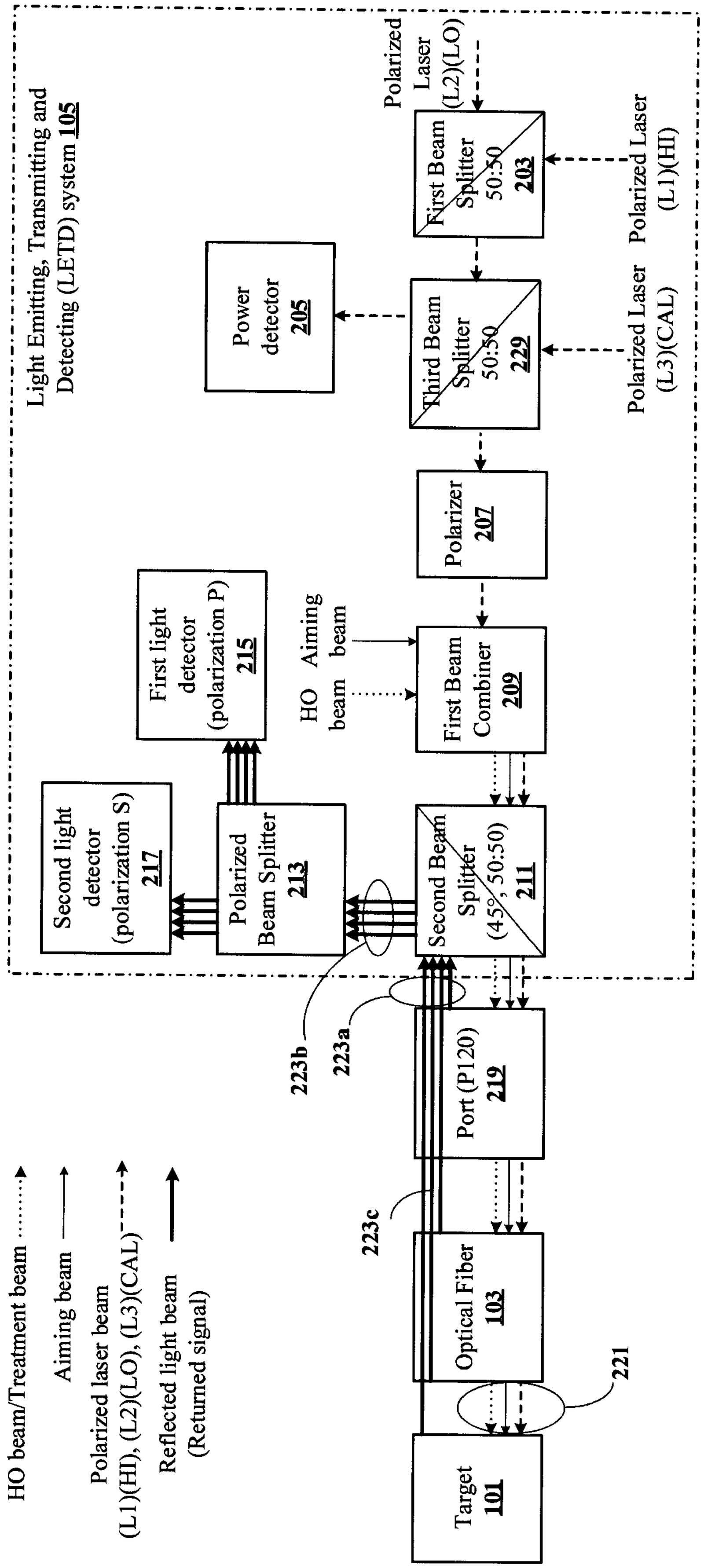
Light Emitting, Transmitting and Detecting (LETD) system 105
Polarized Laser (L2)(LO)
Polarized Laser (L1)(HI)
Polarized Laser (L3)(CAL)
First Beam Splitter 50:50 203
Third Beam Splitter 50:50 229
Power detector 205
Polarizer 207
HO beam
Aiming beam
First Beam Combiner 209
First light detector (polarization P) 215
Second light detector (polarization S) 217
Polarized Beam Splitter 213
Second Beam Splitter (45°, 50:50) 211
223b
223a
Port (P120) 219
223c
Optical Fiber 103
221
Target 101
HO beam/Treatment beam
Aiming beam
Polarized laser beam (L1)(HI), (L2)(LO), (L3)(CAL)
Reflected light beam (Returned signal)

FIG.2D

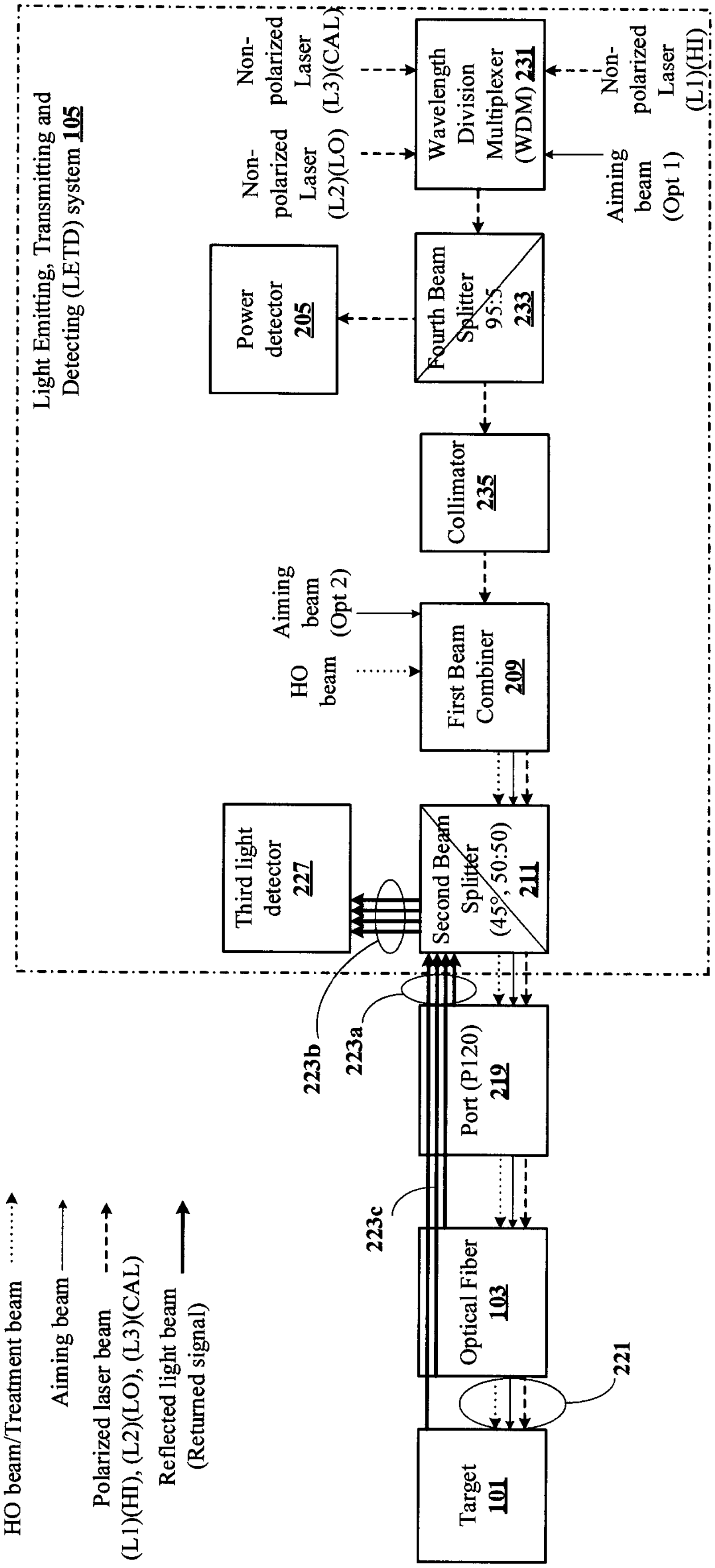

Light Emitting, Transmitting and Detecting (LETD) system 105
Non-polarized Laser (L2)(LO)
Non-polarized Laser (L3)(CAL)
Wavelength Division Multiplexer (WDM) 231
Non-polarized Laser (L1)(HI)
Aiming beam (Opt 1)
Power detector 205
Fourth Beam Splitter 95:5 233
Collimator 235
Aiming beam (Opt 2)
HO beam
First Beam Combiner 209
Third light detector 227
Second Beam Splitter (45°, 50:50) 211
223b
223a
Port (P120) 219
223c
Optical Fiber 103
221
Target 101
HO beam/Treatment beam
Aiming beam
Polarized laser beam (L1)(HI), (L2)(LO), (L3)(CAL)
Reflected light beam (Returned signal)


FIG.2E

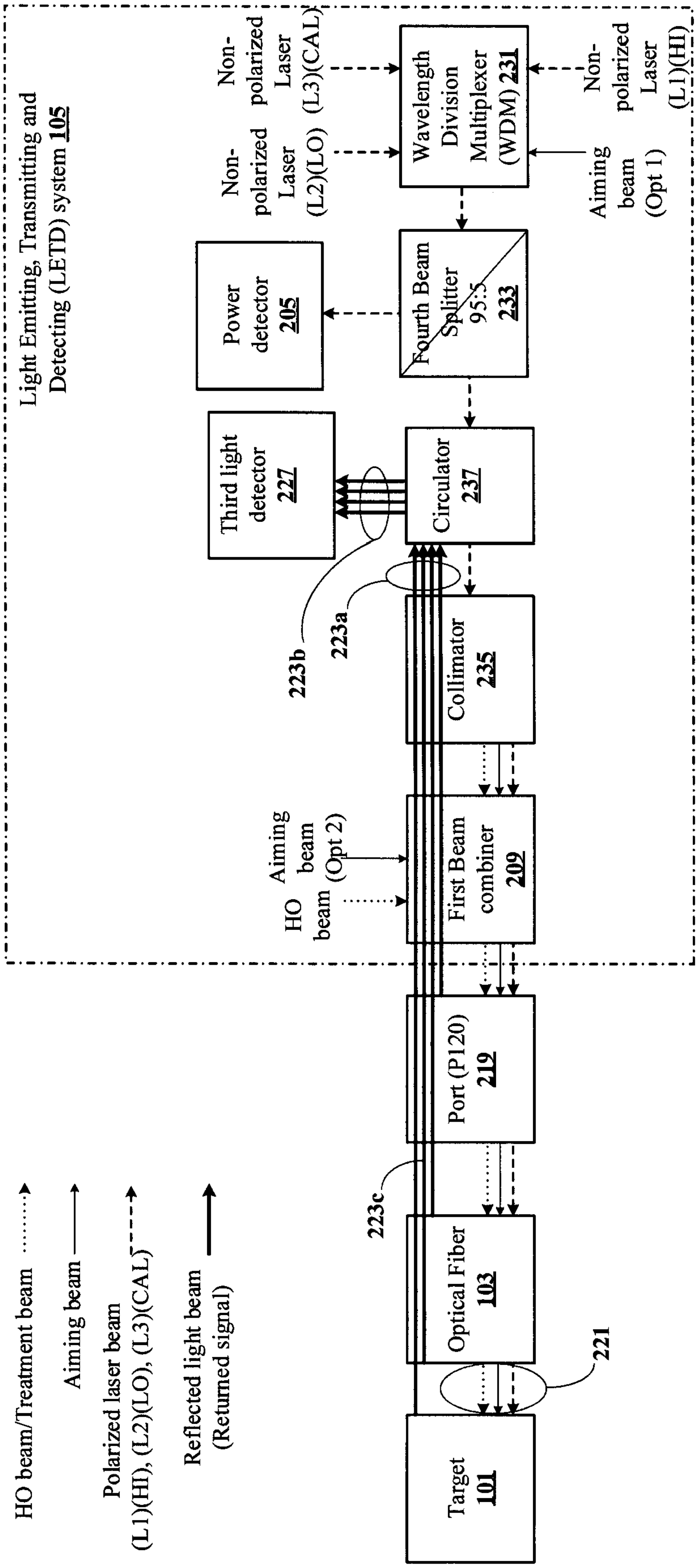
Light Emitting, Transmitting and Detecting (LETD) system 105
Non-polarized Laser (L2)(LO)
Non-polarized Laser (L3)(CAL)
Wavelength Division Multiplexer (WDM) 231
Aiming beam (Opt 1)
Non-polarized Laser (L1)(HI)
Power detector 205
Fourth Beam Splitter 95:5 233
Third light detector 227
Circulator 237
223b
223a
Collimator 235
Aiming beam (Opt 2)
HO beam
First Beam combiner 209
Port (P120) 219
223c
Optical Fiber 103
221
Target 101
HO beam/Treatment beam
Aiming beam
Polarized laser beam (L1)(HI), (L2)(LO), (L3)(CAL)
Reflected light beam (Returned signal)

FIG.2F

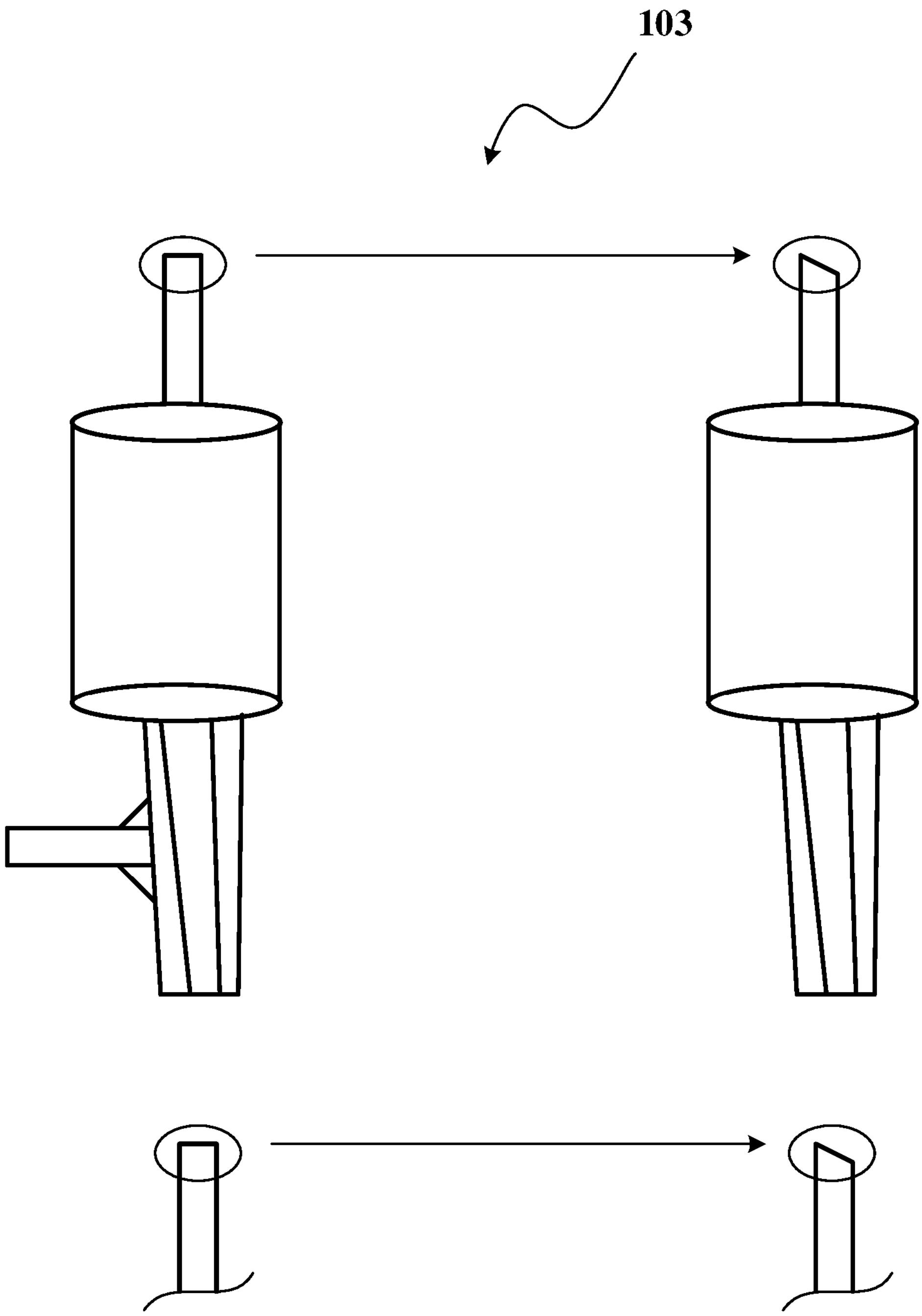


FIG.2G

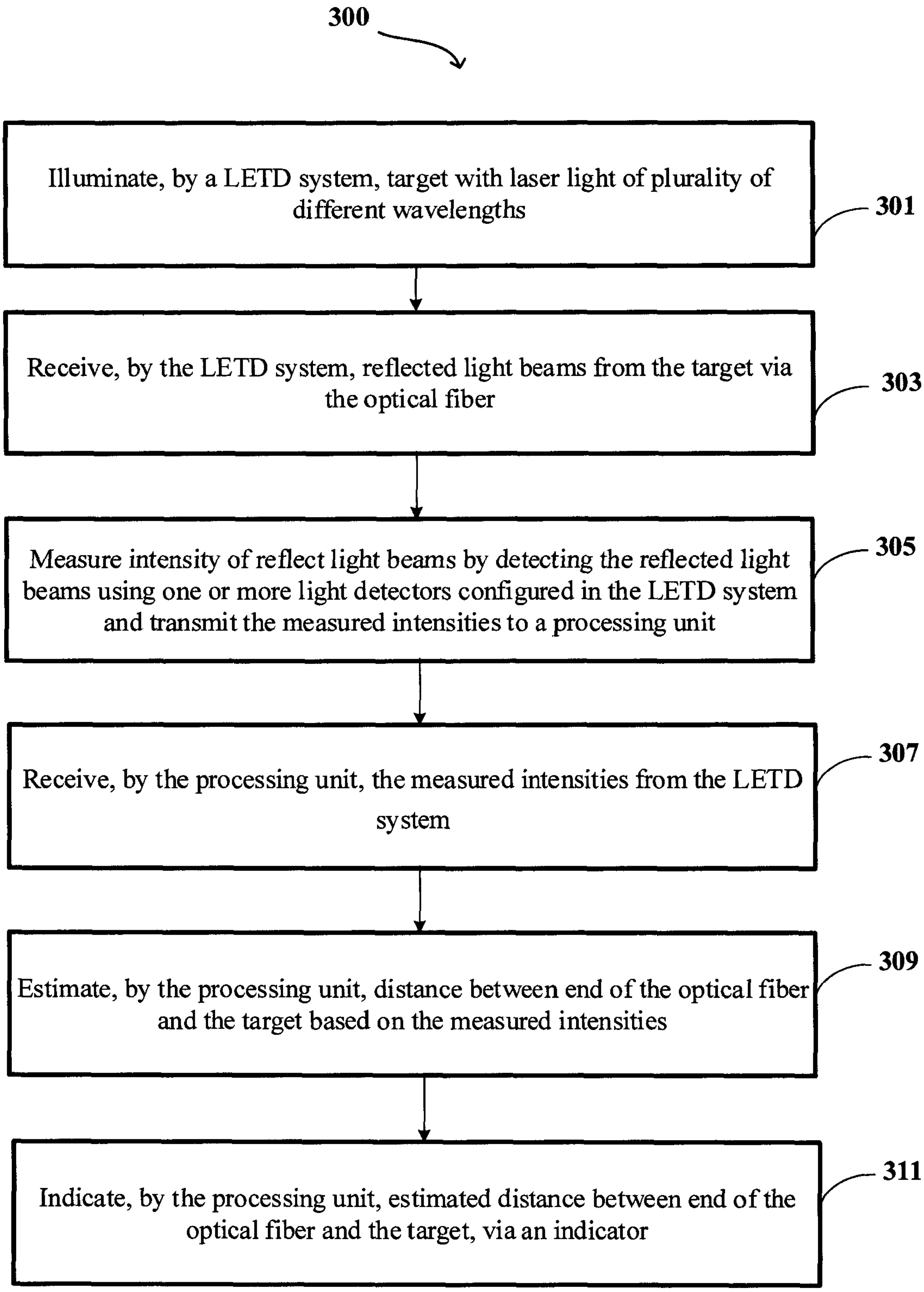
300
Illuminate, by a LETD system, target with laser light of plurality of different wavelengths
301
Receive, by the LETD system, reflected light beams from the target via the optical fiber
303
Measure intensity of reflect light beams by detecting the reflected light beams using one or more light detectors configured in the LETD system and transmit the measured intensities to a processing unit
305
Receive, by the processing unit, the measured intensities from the LETD system
307
Estimate, by the processing unit, distance between end of the optical fiber and the target based on the measured intensities
309
Indicate, by the processing unit, estimated distance between end of the optical fiber and the target, via an indicator
311

FIG.3

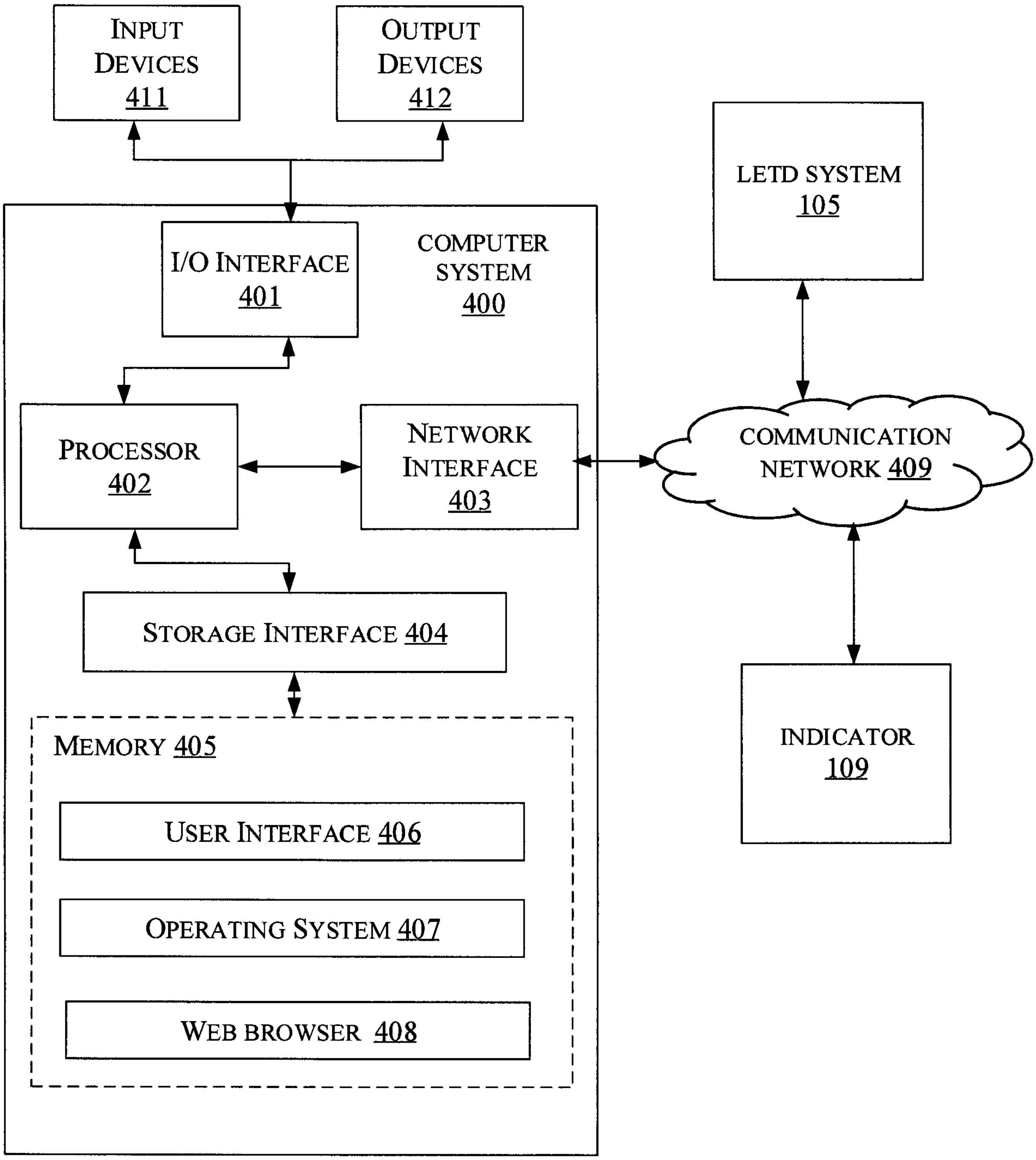
INPUT DEVICES 411
OUTPUT DEVICES 412
LETD SYSTEM 105
I/O INTERFACE 401
COMPUTER SYSTEM 400
PROCESSOR 402
NETWORK INTERFACE 403
COMMUNICATION NETWORK 409
STORAGE INTERFACE 404
MEMORY 405
USER INTERFACE 406
OPERATING SYSTEM 407
WEB BROWSER 408
INDICATOR 109

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63118117